Europäisches Patentamt

European Patent Office

Office européen des brevets

(19)

(11) **EP 1 402 270 B1**

(12) **FASCICULE DE BREVET EUROPEEN**

(45) Date de publication et mention
de la délivrance du brevet:
**29.03.2006 Bulletin 2006/13**

(51) Int Cl.:
***G01N 33/68*** *(2006.01)*     ***G01N 33/50*** *(2006.01)*
***G01N 33/94*** *(2006.01)*

(21) Numéro de dépôt: **02767548.7**

(22) Date de dépôt: **04.07.2002**

(86) Numéro de dépôt international:
**PCT/FR2002/002337**

(87) Numéro de publication internationale:
**WO 2003/005038 (16.01.2003 Gazette 2003/03)**

(54) **IDENTIFICATION DE MODULATEURS DE L'ACTIVITE NEUROTRANSMETTEUR DE L'ACIDE XANTHURENIQUE**

IDENTIFIZIERUNG VON MODULATOREN DES NEUROTRANSMITTERS XANTHURENSÄURE

IDENTIFICATION OF MODULATORS OF NEUROTRANSMITTER ACTIVITY OF XANTHURENIC ACID

(84) Etats contractants désignés:
**AT BE BG CH CY CZ DE DK EE ES FI FR GB GR IE IT LI LU MC NL PT SE SK TR**

(30) Priorité: **05.07.2001 FR 0108937**

(43) Date de publication de la demande:
**31.03.2004 Bulletin 2004/14**

(73) Titulaire: **UNIVERSITE LOUIS PASTEUR**
**67000 Strasbourg (FR)**

(72) Inventeurs:
• **GOBAILLE, Serge**
**F-67550 Vendenheim (FR)**
• **MAITRE, Michel**
**F-67000 Strasbourg (FR)**

(74) Mandataire: **Tezier Herman, Béatrice et al**
**Cabinet BECKER & ASSOCIES**
**25, rue Louis Le Grand**
**75002 Paris (FR)**

(56) Documents cités:
**WO-A-00/73788          WO-A-95/03271**
**WO-A-98/02553          WO-A-98/51290**

• **MILLER DAVID W ET AL: "Role of high-affinity dopamine uptake and impulse activity in the appearance of extracellular dopamine in striatum after administration of exogenous L-DOPA: Studies in intact and 6-hydroxydopamine-treated rats." JOURNAL OF NEUROCHEMISTRY, vol. 72, no. 4, avril 1999 (1999-04), pages 1516-1522, XP008003959 ISSN: 0022-3042**

**Description**

La présente invention concerne des méthodes et compositions pour la sélection et le développement de nouveaux agents pharmacologiques ou de nouveaux médicaments possédant des propriétés ou des mécanismes d'action originaux. La présente invention décrit notamment la caractérisation et l'identification du rôle fonctionnel de plusieurs éléments constitutifs du système nerveux central, jouant un rôle dans le métabolisme ou le transport de l'acide xanthurénique (XA) ou impliqués dans la réception et/ou la transduction du signal médié par cette substance. Ces cibles sont de nouveaux outils pour la définition et la sélection de nouvelles substances chimiques de synthèse, interférant avec ces cibles et modulant les fonctions de XA pour en tirer des profits sur le plan pharmacologique ou médicamenteux. Le domaine d'application de l'invention est, en premier lieu, le traitement d'affections et de maladies neurologiques ou mentales.

L'industrie pharmaceutique est à la recherche continuelle de nouvelles substances naturelles, présentes dans le cerveau, et dont le rôle fonctionnel est important pour la régulation et le contrôle de l'activité psychologique, psychoaffective, mentale, cognitive ou neurologique. La découverte de nouveaux mécanismes dans le domaine de la neurotransmission et de nouvelles cibles participant à ces mécanismes dans le système nerveux sont autant d'outils précieux pour la sélection de nouveaux médicaments dans des indications variées qui seront décrites plus loin. La présente invention décrit pour la première fois le rôle important d'une substance naturelle, l'acide xanthurénique, dans les phénomènes de neurotransmission au niveau central. Ce nouveau système de neurotransmission comporte un système métabolique de synthèse et d'élimination du transmetteur, des transporteurs, des récepteurs et des signaux intracellulaires allumés suite à la stimulation de ces récepteurs. Ces éléments constituent des "cibles" pouvant être modulées par des médicaments potentiels et utilisables pour l'identification, la sélection ou la caractérisation de composés biologiquement actifs. D'autre part, l'invention décrit également différents rôles fonctionnels de l'acide xanthurénique dans le cerveau (interférences avec d'autres neutransmetteurs, rôle dans certains protocoles comportementaux ou neuropharmacologiques) permettant d'envisager des domaines d'applications pharmacologiques et thérapeutiques nouveaux pour cette molécule, ses dérivés ou , plus généralement, les substances naturelles ou de synthèse fixées par les cibles décrites précédemment.

Depuis le début des années 1980, le catabolisme du tryptophane a progressivement occupé un rôle de plus en plus important dans les recherches des causes et des traitements de désordres majeurs au niveau du système nerveux central (Orv. Hetil 1992 Jul 19 ; 133 (29) : 1803 - 1807). Ce sont essentiellement des produits comme l'acide quinolinique et l'acide kynurénique, métabolites du tryptophane, qui ont focalisé l'attention pour leurs profils pharmacologiques. Un déséquilibre dans la formation de ces métabolites, une synthèse ou une dégradation chroniquement altérées, pourraient être responsables de troubles fonctionnels du système nerveux central et des pathologies qui en résulteraient.

S'agissant du XA, autre métabolite du tryptophane, différentes études ont rapporté des effets ponctuels du XA. Ainsi, un effet analgésique a été mentionné pour le XA (50 à 600 mg/kg i.p.) chez le rat Sprague-Dawley, par utilisation des tests de la « plaque chaude » et du « tail flick » (Pharmacol Res. 1998 oct ; 38 (4) : 243 - 250). D'autre part, chez l'animal, l'injection intrapéritonéale de XA a prolongé le temps de latence d'apparition des crises d'épilepsie induites par les injections intracérébroventriculaires d'acide quinolinique, de strychnine ou de pentylènetétrazole. Il semblerait que l'excrétion accrue de XA réponde à un processus de compensation chez les patients manifestant différents stades d'épisodes convulsifs (J. Neural. Transm. 1983; 56 (2-3): 177-185 ; Clin. Endocrinol (Oxf) 1997 Dec ;47 (6): 667 - 677). Après ingestion de tryptophane (5g), l'excrétion urinaire (24h) de XA est nettement augmentée chez les patients déprimés (Psychopharmacology (Berl) 1981 ; 75 (4): 346 - 349; Acta Psychiatr Belg 1979 Nov-Dec ; 79 (6) 638 - 646). D'autres résultats montrent cependant que l'injection ICV de XA, ne modifie pas le tracé EEG chez le rat (Okayama Igakkai Zasshi 104 (5-6) : 471 - 482 1992), que l'injection, chez la souris, de XA n'induit pas de comportement de « head twitches » (Psychopharmacology (Berl) 1977 Mar 16 ;51 (3) : 305 - 309), que le XA ne bloque pas les dépolarisations induites par les acides aminés excitateurs (Brain Res. 1986 Aug. 20 ; 380 (2) : 297 - 302) et que le XA induit l'augmentation de l'expression d'une protéine chaperone du réticulum endoplasmique (Grp 94) et de calreticulin, provoquant une anomalie de conformation de certaines protéines. Les concentrations d'acide xanthurénique nécessaires pour induire ces problèmes en culture de cellules seraient de l'ordre de plusieurs millimolaires (H. Malina; Biochemical and biophysical Research Communications, 265, 600-605 (1999)).

Dans un article de Miller David W et al, il est décrit les conséquences de l'administration de la L-DOPA sur la dopamine. Y sont étudiés en particulier les effets comportementaux et neurochimiques de cette administration (Miller David W et al. : Journal of neurochemistry, vol., 72, no. 4, avril 1999, pp. 1516-1522). WO 9503271 et WO 9802553 décrivent des inhibiteurs de kynéninase et/ou kynurénine-3-hydroxylase utilisables pour traiter des maladies neurodégénératives. Ces demandes de brevets décrivent aussi des méthodes d'identification de composés en les testant sur une préparation membranaire ou des oocytes exprimant l'enzyme kynurénine-3-hydroxylase (enzyme de synthèse de XA) et en mettant en évidence un effet biologique. L'exemple 5 de la demande WO 9851290 spécifie l'effet anesthésiant de l'association de la tetrodotoxine avec l'épinéphrine et/ou la bupivacaine. WO 0073788 divulgue des méthodes d'utilisation de la gabapentine pour identifier des agents modulateurs du récepteur GABA$_B$.

Toutefois, à ce jour, le rôle du XA demeure non caractérisé, son mode d'action et son rôle physiologique restent inconnus, l'existence d'un récepteur éventuel n'a pas été démontrée, et aucune stratégie thérapeutique basée sur cette molécule n'a été entreprise.

La présente invention résulte de la description, la caractérisation et l'identification du rôle fonctionnel de plusieurs éléments constitutifs du système nerveux central, jouant un rôle dans le métabolisme ou le transport de l'acide xanthurénique (XA) ou impliqués dans la réception et/ou la transduction du signal médié par cette substance. Ces cibles sont de nouveaux outils pour la définition et la sélection de nouvelles substances chimiques de synthèse, interférant avec ces cibles et modulant les fonctions de XA pour en tirer des profits sur le plan pharmacologique ou médicamenteux. L'invention démontre plus particulièrement le rôle du XA dans le système nerveux central et son implication, en tant que neurotransmetteur, dans de multiples mécanismes physio-pathologiques. L'invention montre notamment :

- l'existence et les caractéristiques de la libération extracellulaire du XA dans le cortex,
- l'existence d'un site de liaison de haute affinité pour cette substance dans le cerveau, modulé par les dérivés adényliques (adénosine, ADP, ATP), les ions cuivre et zinc, et l'acide kynurénique et ses dérivés,
- l'existence d'interrelations fonctionnelles in vivo entre le XA et la dopamine cérébrale, permettant notamment de présenter un rôle pour les terminaisons XA du cortex frontal dans la régulation du système dopaminergique, en particulier via un impact sur les activités glutamatergiques et GABAergiques. Le XA provoque des stéréotypies doses-dépendantes, de nature dopaminergique, lorsqu'il est administré dans le noyau A$_{10}$, ou dans le nucleus accumbens, le striatum, les ventricules latéraux. Ceci permet d'impliquer le système XA comme une cible importante pour des ligands des sites récepteurs correspondants, ces ligands régulant les activités glutamate / GABA / dopamine impliquées dans la production des symptômes schizophréniques. On peut donc prévoir que des ligands des récepteurs XA possèdent un intérêt important pour le traitement de certains symptômes psychotiques,
- l'induction d'effets électrophysiologiques par le XA en quantités micromolaires sur certaines conductances ioniques membranaires de cellules exprimant les récepteurs XA,
- la mise en évidence d'un transport actif sodium-dépendant de XA par des neurones en cultures. Cette fonction représente une nouvelle cible pour des analogues structuraux de XA, ou plus généralement pour des ligands synthétiques interférant avec ce système de transport. En effet, l'inhibition de ce système de transport peut avoir pour conséquence l'augmentation de la durée de vie du XA dans certains compartiments fonctionnels du cerveau, en particulier le compartiment synaptique. Les inhibiteurs de ce transport peuvent donc représenter une nouvelle classe de substances possédant des effets bénéfiques dans plusieurs types de pathologies neurologiques ou mentales,
- les réactions au stress aigu entraînent non seulement une libération extracellulaire de noradrénaline, mais également une forte libération de XA. La libération de cette dernière substance participe à la mise en place des réactions d'adaptation à ce stress. Les résultats indiquent donc que les ligands modulant l'activité du système XA peuvent avoir des effets utilisables chez l'homme pour la régulation des réactions au stress.
- la capacité d'agonistes synthétiques de XA d'induire un effet électrophysiologique du type de XA sur les cellules NCB-20 et, en particulier, de moduler la dopamine cérébrale in vivo,
- la capacité d'antagonistes du XA, tels le NCS-486, d'antagoniser les effets électrophysiologiques induits par le XA ou ses agonistes, et en particulier d'inhiber l'activité dopaminergique du XA in vivo, et
- les effets neuropharmacologiques in vivo spécifiques du XA, en application locale ou en administration systémique, qui induit sédation, anxiolyse, des effets dopaminergiques (exploitables dans plusieurs domaines tels que addiction aux drogues, maladie de parkinson, schizophrénie, troubles de l'humeur, etc.), des effets antidépresseurs et des effets bénéfiques sur la mémoire et sur les interactions sociales.

La présente demande démontre ainsi que le XA, pour lequel aucun rôle fonctionnel n'était connu jusqu'a présent dans le CNS (pour « Central Nervous System » ou encore « Système Nerveux Central »), possède des propriétés largement en faveur d'un rôle de neurotransmetteur / neuromodulateur. Ce nouveau système de neurotransmission / neuromodulation représente un éventail de cibles potentielles (enzymes de synthèse et de dégradation de XA, sites récepteurs, sites de transport, sites de libération) pour des ligands ou composés, de synthèse ou d'origine naturelle, modulant l'activité de XA et représentant, de ce fait, des médicaments potentiels dans plusieurs domaines de la thérapeutique neurologique et mentale.

La présente demande vise à identifier un composé modulant l'activité de l'acide xanthurénique pour la préparation d'un médicament destiné au traitement de pathologies du système nerveux. Selon des variantes préférées de mise en oeuvre, l'invention a plus particulièrement pour objet :

Un composé modulant spécifiquement l'activité de l'acide xanthurénique pourrait être utilisé pour la préparation d'un médicament destiné au traitement de pathologies du système nerveux, et dans une méthode de traitement correspondante. Le composé est dit spécifique lorsqu'il module d'abord l'activité du XA (e.g., son transport, son métabolisme ou l'activité du/des récepteurs XA) sans affecter directement, en première intention, de façon significative l'activité d'autres systèmes de neurotransmission / neuromodulation centrale. Bien entendu, la modulation du système XA central entraîne des répercussions et des adaptations dans d'autres systèmes, en particulier le système dopaminergique ou GABAergique, mais pas de manière substantielle dans le système sérotoninergique. Des composés spécifiques particuliers selon l'invention sont des composés capables de lier préférentiellement un récepteur naturel XA sans lier, de manière spécifique ou substantielle, un récepteur sérotoninergique ou autre récepteur d'autres neurotransmetteurs/ neuromodulateurs.

Comme indiqué ci-dessus, les composés utilisés sont préférentiellement des modulateurs spécifiques de l'activité XA.

Un composé modulant l'activité de l'acide xanthurénique pourrait être utilisé pour la préparation d'un médicament destiné au traitement de maladies neurodégénérattves, notamment de la maladie de Parkinson, d'Alzheimer ou de l'ALS, ou de maladies mentales, telles que la schizophrénie, ou encore de la dépendance à certaines drogues, notamment aux opiacées.

Dans le contexte de la présente demande, le terme « modulation » désigne soit une stimulation, soit une inhibition de l'activité XA. La stimulation peut être obtenue par l'utilisation de composés mimant l'activité de XA ou stimulant la quantité ou l'efficacité de XA. L'inhibition peut être partielle (e.g., une réduction de l'activité). Elle peut être obtenue en diminuant la quantité de XA présente ou en inhibant la capacité de XA à produire un effet biologique, ou en bloquant ou inhibant l'effet biologique induit par XA.

De ce fait, au sens de la présente demande, le terme « activité de l'acide xanthurénique » désigne notamment son interaction avec un récepteur c'est-à-dire l'activité du système utilisant le XA comme médiateur. Il s'agit d'un composé modulant l'activité du site récepteur XA en agissant sur un site régulateur ou allostérique de ce récepteur.

Le composé utilisé est un composé modulant la liaison de XA à son récepteur membranaire naturel (e.g., un agoniste ou un antagoniste). Un agoniste est un composé présentant une affinité pour le site de liaison du XA, et produisant un signal analogue à celui produit par le XA. Un antagoniste est un composé présentant une affinité pour le site de liaison du XA, et empêchant la liaison et la production d'un signal par le XA ou un agoniste du XA. De tels composés peuvent être des analogues de XA, des anticorps anti-XA, des composés de synthèse, etc. Ainsi, un composé modulant la liaison de XA à un récepteur membranaire permet d'impliquer le système XA comme une cible importante pour des ligands des sites récepteurs correspondants, ces ligands régulant les activités glutamate / GABA / dopamine impliquées dans la production des symptômes schizophréniques. Les ligands des récepteurs XA possèdent un intérêt particulier pour le traitement de certains symptômes psychotiques. Les composés antagonistes du récepteur XA trouvent plus spécifiquement des applications dans la régulation de l'éveil comportemental et dans certaines pathologies mentales ou une hyperdoparninergie. De tels composés peuvent être identifiés, synthétisés, ou caractérisés par des méthodes décrites ci-après.

Les composés modulant l'activité du XA selon l'invention peuvent être de nature et d'origine variées. Il peut s'agir des produits inorganiques ou organiques et notamment d'un polypeptide (ou une protéine ou un peptide), d'un acide nucléique, d'un lipide, d'un polysaccharide, d'un composé chimique ou biologique, etc. Le composé peut être d'origine naturelle ou synthétique et issu notamment d'une banque combinatoire. De préférence, ces composés sont des dérivés de l'acide xanthurénique ou de l'acide kynurénique.

Les composés ou compositions selon l'invention pourraient être administrés de différentes manières et sous différentes formes. Ainsi, ils peuvent être injectés par voie systémique ou orale, de préférence systémique, comme par exemple par voie intraveineuse, intra-musculaire, sous-cutanée, trans-dermique, intra-artérielle, intracérébrale, intra-péritonéale, intracérébrovasculaire, etc. Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc. Les composés peuvent également être administrés sous forme de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

A titre indicatif, les résultats présentés dans les exemples montrent que le XA exerce un effet sédatif lorsqu'il est administré chez l'animal à des doses supérieures à environ 50 mg/kg. Les doses préférées pour obtenir un effet anxiolytique chez l'animal sont des doses inférieures à 50 mg/mg. Un effet anti-dépresseur est avantageusement obtenu chez l'animal à des doses comprises entre 100 et 200 mg/kg.

L'invention concerne également des méthodes pour identifier, sélectionner ou caractériser des composés biologiquement actifs, notamment de composés actifs sur le système nerveux, lesdites méthodes étant basées sur la modulation de l'activité du XA. Les méthodes de l'invention peuvent comprendre des tests de liaison in vitro, des tests de liaison en système cellulaire (ou sur des préparations membranaires, naturelles ou synthétiques) ou des tests fonctionnels en système cellulaire ou artificiels.

La méthode de sélection, identification ou caractérisation de composés selon l'invention comprend la mise en contact d'un composé test avec une cellule (ou une préparation membranaire naturelle ou synthétique) exprimant un récepteur naturel de XA impliquée dans l'activité de à l'acide xanthurénique, et la mise en évidence d'une liaison du composé test à ladite molécule.

La molécule cible est le récepteur du XA naturel.

La liaison du composé test à la molécule cible peut être mise en évidence par exemple en utilisant un composé test marqué ou en utilisant un ligand marqué de la cible moléculaire (par exemple un ligand marqué du récepteur XA ou un anticorps marqué spécifique d'une enzyme de synthèse du XA ou d'un transporteur du XA), le déplacement de la liaison du ligand marqué reflétant la liaison du composé test. Le ligand marqué peut être tout produit liant la molécule cible (anticorps, agoniste ou antagoniste, fragment ou dérivé d'un ligand endogène, etc.). Le ligand peut être marqué par toute technique connue de l'homme du métier, notamment par incorporation d'un élément radioactif, fluorescent, luminescent, enzymatique, colorimétrique, etc.

Dans un mode de réalisation particulier, le ligand est un anticorps marqué, spécifique de la cible moléculaire considérée. L'anticorps peut être polyclonal ou monoclonal. Il peut également s'agir d'un fragment ou dérivé d'anticorps, comme par exemple des fragments Fab, Fab'2, CDR, etc. Les anticorps peuvent être produits de manière conventionnelle, par immunisation la cible moléculaire (ou une partie immunogène de celle-ci), et récupération du sérum (polyclonal) ou des cellules de la rate (pour fabriquer des hybridomes par fusion avec une lignée appropriée), comme décrit par exemple dans Vaitukaitis et al. (J Clin Endocrinol Metab. 1971, 33(6), 988-91) ou dans Harlow et al. (Antibodies: A laboratory Manual, CSH Press, 1988). Les fragments Fab ou F(ab')2 peuvent être produits par exemple selon Riechmann et al., 1988, Nature 332, 323-327.

La cible moléculaire est le récepteur du XA naturel et le ligand est un ligand endogène, un agoniste ou un antagoniste du récepteur, marqué. Dans un mode de mise en oeuvre spécifique, on utilise du XA marqué, notamment radiomarqué, en particulier tritié.

Selon un mode particulier, la méthode comprend donc la mise en contact d'un composé test avec une cellule (ou une préparation membranaire naturelle ou synthétique) exprimant un récepteur à l'acide xanthurénique naturel et la mise en évidence d'une liaison du composé test au récepteur.

Selon un autre mode particulier, la méthode comprend la mise en contact d'un composé test avec une cellule (ou une préparation membranaire naturelle ou synthétique) exprimant une molécule cible impliquée dans l'activité de l'acide xanthurénique, en présence d'un ligand marqué de ladite cible, et la mise en évidence d'une liaison du composé test par détermination du déplacement de la liaison du ligand marqué.

Dans une autre variante, le procédé de l'invention comprend la mise en contact d'un composé test avec une cellule exprimant une molécule cible impliquée dans l'activité de l'acide xanthurénique (en particulier le récepteur de l'acide xanthurénique), et la mise en évidence d'un effet biologique ou pharmacologique caractéristique d'une liaison du composé test à ladite molécule cible. L'effet biologique ou pharmacologique peut être l'expression d'une ou plusieurs protéines cellulaires, l'expression d'un récepteur, l'activation d'un gène, l'internalisation du récepteur, l'apparition d'un courant électrique, d'un flux ou d'un influx d'ions, etc. La mise en évidence de cet effet peut être réalisée par tous moyens appropriés, comme la mesure de l'expression d'un gène rapporteur, la mesure de l'expression membranaire d'un récepteur, le dosage des ions ou d'un courant électrique, etc.

Classiquement, l'effet du composé test est comparé à l'effet déterminé en l'absence dudit composé. En outre, l'effet du composé test peut être déterminé en présence d'un ligand du récepteur XA, par exemple du XA lui-même, notamment lorsqu'une activité de modulation ou d'inhibition de XA est recherchée.

Selon une variante particulière, le procédé de l'invention comprend donc la mise en contact d'un composé test avec une cellule exprimant un récepteur à l'acide xanthurénique, en présence d'un ligand dudit récepteur, la mesure d'un effet biologique ou pharmacologique caractéristique d'une liaison au récepteur XA et la comparaison de l'effet mesuré à celui obtenu en l'absence de composé test. Un tel procédé est particulièrement adapté à la recherche, la sélection, la caractérisation ou l'amélioration de composés inhibant l'activité de XA.

Les cellules utilisées dans les tests peuvent être toutes cellules exprimant une molécule cible impliquée dans l'activité du XA, notamment un récepteur XA. Il peut s'agir de cellules exprimant naturellement cette molécule (e.g., ce récepteur), ou de cellules modifiées génétiquement ou traitées pour sur-exprimer ladite molécule (e.g., ledit récepteur). Il s'agit, dans un mode préféré de l'invention, de cellules de mammifères (cellules nerveuses, hépatocytes, fibroblastes, cellules endothéliales, musculaires, etc.). De manière encore plus préférée, ces cellules sont des cellules humaines. Il peut également s'agir de cultures primaires ou de lignées établies. Dans un autre mode de mise en oeuvre, il est possible également d'utiliser des cellules procaryotes (bactéries), des cellules de levure (Saccharomyces, Kluyveromyces, etc.), des cellules végétales, etc.

Dans un mode préféré, on utilise des cellules d'origine nerveuse ou synaptique, notamment des neurones, des cellules gliales, des astrocytes, du matériel d'origine synaptique (membranes, synaptosomes, synaptoneurosomes), etc. Ces cellules peuvent être isolées, cultivées et caractérisées selon des techniques connues, décrites dans les exemples.

La présente demande montre, pour la première fois, l'existence d'un récepteur de haute affinité pour le XA exprimé dans différentes populations cellulaires ou régions du cerveau. Ce récepteur présente les caractéristiques pharmacologiques suivantes :

- expression dans le cerveau,
- Kd du récepteur synaptique pour le XA inférieur ou égal à environ 300 nM à 1300 nM,
- récepteur modulé par les dérivés adényliques (adénosine, ADP, ATP)
- récepteur modulé par les ions cuivre et zinc,
- récepteur modulé par l'acide kynurénique,
- l'activation du récepteur par le XA induit un courant électrique,
- le récepteur est exprimé par les cellules NCB-20.

Par ailleurs, des indications électrophysiologiques et biochimiques laissent prévoir un récepteur couplé à des G protéines, sans que cela ait été formellement démontré.

La caractérisation de ce récepteur, son identification et la description de procédés permettant de mesurer la liaison de molécules sur ce récepteur permettent, selon la présente demande, de mettre en évidence de nouveaux composés biologiquement actifs, capables de moduler l'activité du XA. La démonstration du rôle pharmacologique de XA souligne l'importance de la mise à disposition de tels composés actifs.

Dans un mode particulier, pour la mise en oeuvre des procédés ci-dessus, on utilise une préparation membranaire exprimant un récepteur du XA. La préparation membranaire peut être, avantageusement, d'origine naturelle, c'est-à-dire produite à partir d'une cellule exprimant le récepteur. On peut produire des préparations membranaires par lyse mécanique, chimique, physique, électrique, etc., et notamment par traitement avec des détergents, ultrasons, congélation/décongélation, etc., comme illustré dans les exemples. Cette préparation membranaire se caractérise essentiellement par la présence de fragments de membrane, comprenant une bicouche lipidique dans laquelle tout ou partie du récepteur XA est présente. Ces préparations membranaires sont généralement dépourvues de cellule intacte. En outre, elles peuvent être enrichies en débris membranaires par des traitements appropriés (centrifugation, etc.). On peut également utiliser une préparation membranaire d'origine synthétique, comme par exemple un liposome dans lequel tout ou partie du récepteur XA a été introduit, ou une membrane supportée.

Pour les tests de liaison ou fonctionnels ci-dessus, les composés peuvent être mis au contact des cellules (ou des préparations membranaires) à différents moments, selon leur(s) effet(s), leur concentration ou la nature des cellules et pour des périodes variées, qui peuvent être ajustées par l'homme du métier. Le test peut être effectué sur tout support approprié et notamment sur une plaque, une lame, une boîte, dans un tube ou une flasque. Généralement, la mise en contact est réalisée dans une plaque multi-puits ce qui permet de conduire, en parallèle, des essais nombreux et variés. Parmi les supports typiques on trouve des plaques de microtitration et plus particulièrement des plaques 96 ou 384 puits (ou plus), faciles à manipuler.

Selon le support et la nature du composé test, des quantités variables de cellules peuvent être utilisées lors de la mise en oeuvre des méthodes décrites.

De manière classique, $10^2$ à $10^6$ cellules sont mises en contact avec un type de composé test, dans un milieu de culture approprié, et de manière préférentielle entre $10^3$ et $10^5$ cellules. Lorsqu'une préparation membranaire est utilisée, on applique généralement 0,01 à 50 mg de protéine par essai, plus préférentiellement de 0,05 à 2 mg de protéine par essai. Les essais peuvent être réalisés dans tout type de milieu approprié, comme par exemple des solutions salines, des tampons, etc. On peut citer notamment des tampons Tris, Pipes, Hepes, etc. La température est, typiquement, proche de la température ambiante. Le pH du milieu est avantageusement compris entre 5,5 et 8, plus préférentiellement entre 7 et 8. Il est entendu que ces paramètres peuvent être ajusté par l'homme du métier, suivant les indications fournies dans les exemples.

La quantité (ou la concentration) de composé test peut également être ajustée par l'utilisateur selon le type de composé (sa toxicité, sa capacité de pénétration cellulaire, etc.), la longueur de la période d'incubation, etc. Généralement, les cellules (ou membranes) sont exposées à des quantités de composés test qui varient de 1 nM à 1 mM. Il est bien sûr possible de tester d'autres concentrations sans dévier de la présente invention. Chaque composé peut, de plus, être testé, en parallèle, à différentes concentrations et sur différentes périodes. Par ailleurs, des adjuvants et/ou vecteurs et/ou produits facilitant la pénétration des composés dans les cellules tels que des liposomes, des lipides cationiques, des polymères, des peptides issus de virus, etc., peuvent, en outre, être utilisés, si nécessaire. Le contact peut être maintenu pendant une période comprise entre 1 minute et plusieurs heures, selon la nature de la molécule cible. Lorsque la molécule cible est le récepteur XA, le contact est maintenu typiquement pendant moins de 1 heure environ. Lorsque la molécule cible est une molécule intracellulaire, le contact peut être maintenu plus longtemps.

Dans une autre variante, l'invention est basée sur des tests de liaison in vitro entre le XA et un composé test ou entre un composé test et une molécule cible (par exemple tout ou une partie du récepteur XA). Selon ces modes de mise en oeuvre, le procédé pour la sélection, l'identification ou la caractérisation de composés actifs comprend:

- la mise en contact d'un composé test avec une molécule cible impliquée dans l'activité du XA, par exemple une enzyme de synthèse ou de régulation, un transporteur, un récepteur, ou un fragment de ceux-ci, et
- la détermination de la liaison éventuelle dudit composé test sur ladite molécule.

La liaison du composé test peut être mise en évidence de différentes manières, comme par exemple par migration sur gel ou électrophorèse des complexes formés. D'autres méthodes basées sur la luminescence ou utilisant la technique FRET (Fluorescence Resonance Energy Transfer) bien connue de l'homme du métier ou la technique SPA (« Scintillation Proximity Assay »), peuvent être mises en oeuvre, dans le cadre de la présente invention, pour déterminer la liaison éventuelle du composé test sur la molécule cible. Lorsque la cible est un récepteur, la mesure de la liaison peut être effectuée en présence d'un ligand marqué, par détermination de la déplacement du ligand par le composé test.

La présente invention peut être appliquée à tout type de composé test. Ainsi, le composé test peut être tout produit qui se présente sous une forme isolée ou en mélange avec d'autres produits. Le composé peut être défini en termes de structure et/ou de composition ou ne pas être défini. Le composé peut, par exemple, être un produit isolé et structurellement défini, un produit isolé de structure indéfinie, un mélange de produits connus et caractérisés ou une composition indéfinie comprenant un ou plusieurs produits. De telles compositions indéfinies peuvent être, par exemple, des échantillons de tissus, des fluides biologiques, des surnageants cellulaires, des préparations végétales, etc. Les composés test peuvent être des produits inorganiques ou organiques et notamment un polypeptide (ou une protéine ou un peptide), un acide nucléique, un lipide, un polysaccharide, un composé chimique ou biologique tel qu'un facteur nucléaire, un cofacteur ou tout mélange ou dérivé de ces derniers. Le composé peut être d'origine naturelle ou synthétique et inclure une banque combinatoire, un clone ou une banque de clones d'acides nucléiques exprimant un ou plusieurs polypeptide(s) liant l'ADN, etc.

D'autres avantages et aspects de l'invention apparaîtront à la lecture des exemples qui suivent, qui doivent être considérés comme illustratifs et non limitatifs.

**LEGENDE DES FIGURES**

| | |
|---|---|
| **Figure 1** : | Concentration extracellulaire de XA au niveau du Cortex préfrontal (CPF) |
| **Figure 2A et 2B :** | Libération de XA dans le CPF après stimulation électrique du VTA (figure 2A = 100 $\mu$A ; figure 2B = 200 $\mu$A). Dopamine = triangles noirs ; XA = carrés noirs |
| **Figure 3 :** | Libération de XA induite par 100 mM KCl dans la sonde pendant 5 min. |
| **Figure 4 :** | Libération de XA induite par la présence de 50 $\mu$M de veratridine dans la sonde pendant 20 minutes. |
| **Figure 5 :** | Libération de XA dans le CPF sous stimulation électrique (barres blanches). La même expérience, reproduite en l'absence d'ions calcium et en présence d'EGTA, ne montre plus de libération accrue de XA sous stimulation électrique (barres noires). |
| **Figure 6 :** | Libération de XA dans le CPF après stimulation électrique du VTA (barres blanches, libération de base = 21 pmol / 5 min). Même expérience mais en présence de 2.0 $\mu$M de TTX dans le liquide de dialyse (barres noires, libération de base = 7 pmol / 5 min). En présence de TTX, il n'existe plus de libération induite de XA. |
| **Figure 7:** | Répartition de XA dans le cerveau de rat dans les conditions physiologiques. Concentrations selon un code couleur arbitraire. Abréviations : CxPrFr : cortex préfrontal ; CxFR : cortex frontal ; CxPar : cortex pariétal ; CxOcc : cortex occipital ; C : cervelet ; GP : globus pallidus ; CP : caudé putamen ; n.Acc : noyau accumbens ; TO : tubercules olfactifs ; BO : bulbes olfactifs ; S : septum ; Hb : habenula ; Hi : hippocampe ; Th : thalamus ; Hy : hypothalamus ; SN : substance noire (A9) ; VTA : aire tegmentale ventrale (A10) ; Rad : noyau raphé dorsal ; Ram : noyau raphé médian ; LC : locus coeruleus (A6) ; MO : médulla oblongata. |
| **Figure 8 :** | Effet du pH sur la liaison de l'AX sur son (ses) site(s) de liaison |
| **Figure 9 :** | Etude de la linéarité du binding spécifique en fonction de la concentration en protéine. |
| **Figure 10 :** | Détermination de la constante d'association $K_{on}$ |
| **Figure 11 :** | Détermination de la constante de dissociation $K_{off}$ |
| **Figure 12 :** | Courbe de saturation des sites membranaires de XA. Détermination du Kd et du Bmax. |
| **Figure 13 :** | Inhibition compétitive de la fixation de XA radioactif par XA non marqué. Détermination des IC 50 pour l'acide xanthurénique |

**Figure 14 :**     Influence d'ions métalliques sur la liaison de l'acide xanthurénique à son (ses) site(s) de liaison

**Figure 15 :**     Effet de la variation du pH sur la liaison de l'AX à son (ses) site(s) de liaison en présence d'ions cuivre.

**Figure 16**     :Etude de la linéarité du binding spécifique en fonction de la concentration protéique en présence d'ions cuivre

**Figure 17 :**     Effet/dose du Cuivre ($Cu^{2+}$) sur la liaison de l'AX à son (ses) site(s) de liaison

**Figure 18**     : Mesure de la courbe de saturation et des paramètres Kd et Bmax en présence d'ions cuivre.

**Figure 19 :**     Inhibition compétitive de XA marqué par XA non radioactif. Détermination des IC 50 pour l'acide xanthurénique.

**Figure 20 :**     Dose / effet du XA sur la réponse dopaminergique après injection locale dans le CPF.

**Figure 21**     : Evolution des taux de dopamine, DOPAC et HVA dans le CPF après application locale de 20 $\mu$M de XA.

**Figure 22:**     Effets de la présence de l'antagoniste NCS-486 sur la libération extracellulaire de dopamine après application locale de 20 $\mu$M de XA dans le CPF.

**Figure 23**     : Stratégie expérimentale dans les expériences de patch-clamp.

**Figure 24**     : Effet de l'acide xanthurénique sur les cellules NCB-20.

    A. Traces types enregistrées à partir d'un fragment de membrane en conditions contrôle et pendant l'application de l'acide xanthurenique à une concentration de 20 $\mu$M. Le fragment de membrane était stimulé par une rampe de potentiel comme indiqué par le protocole illustré par la trace du bas. En présence de l'acide xanthurénique un grand courant sortant se développe aux potentiels positifs. La trace a été numérisée à une fréquence de 2 kHz.

    B. Evolution du courant sortant moyen mesuré à un potentiel de 95 mV. En présence de l'acide Xanthurénique ce courant est augmenté de manière considérable.

    C. Relation courant-potentiel du courant active en présence de l'acide xanthurénique (après soustraction du courant obtenu en condition contrôle). La courbe en trait continu est l'ajustement du modèle de Boltzmann à ce courant

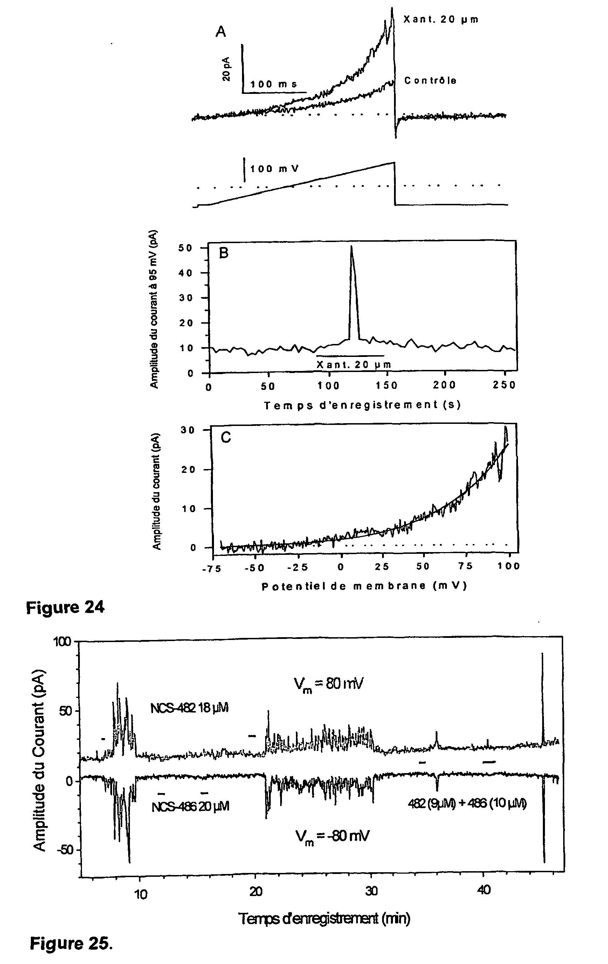

**FIGURE 25.**     Pharmacologie de la réponse à l'acide xanthurénique.

    Le courant membranaire mesuré aux potentiels de -80 et 80 mV est représenté en fonction du temps d'enregistrement. La période d'application des ligands du récepteur du xanthurénate est matérialisé par les traits pleins : en haut et en bas respectivement pour le NCS-482 et le NCS-486 seuls et au milieu pour le mélange des deux aux concentrations indiquées.

    Le NCS-482 et le NCS-486 sont des dérivés de l'acide xanthurénique.

    Le NCS-486 seul ne change pas l'amplitude du courant.

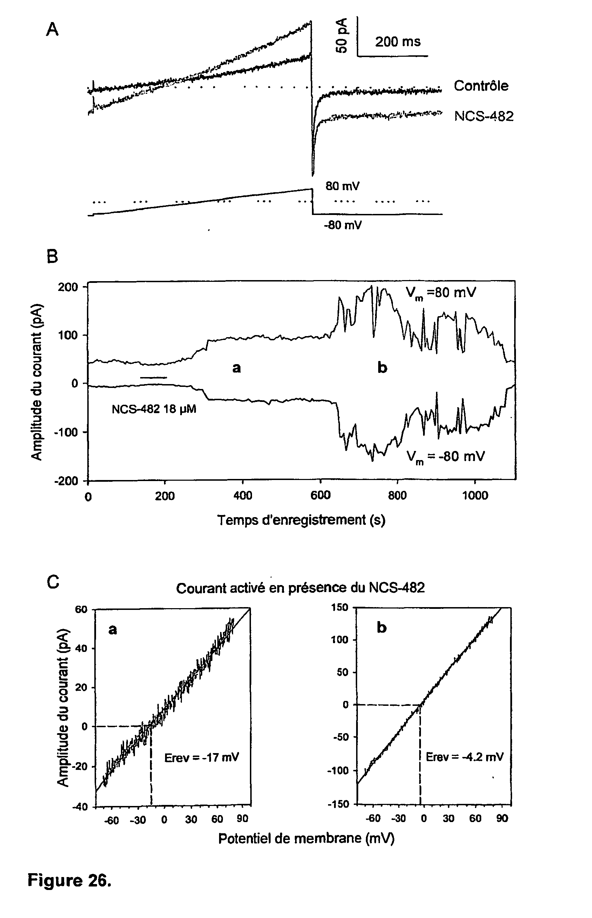

**Figure 26:**     Activation d'une conductance chlorure par le récepteur de l'acide xanthurénique. L'agoniste utilisé dans cette expérience est le NCS-482 à une concentration de 20 $\mu$M. La réponse se développe en deux phases (a et b). Le nature du courant dans ces deux phases est différentes comme le montre le potentiel d'inversion du courant obtenu dans chacune des deux phases (diagrammes du bas).

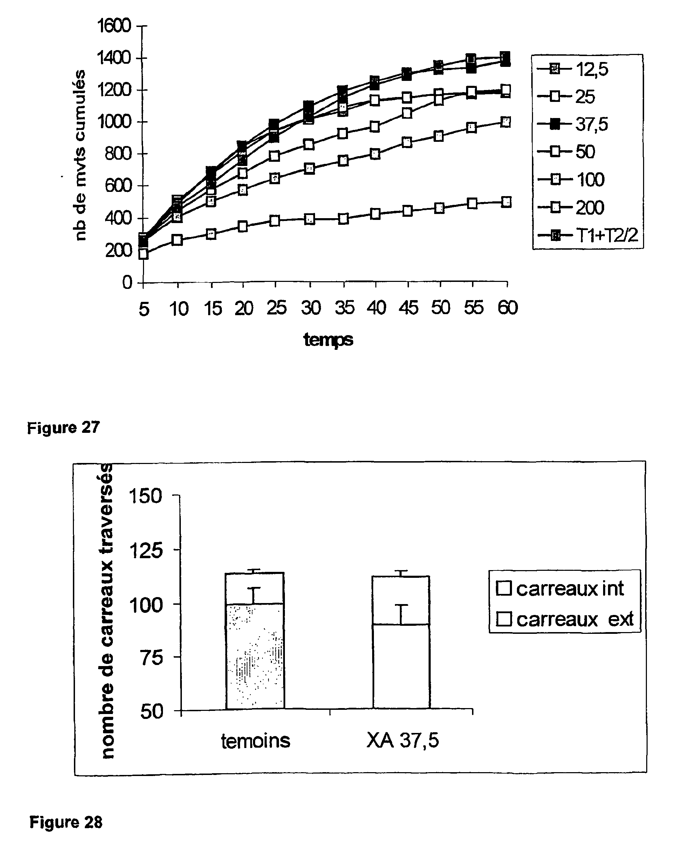

**Figure 27:**     Activité locomotrice spontanée des animaux traités par des doses croissantes de XA. Le temps est mesuré en minutes.

**Figure 28 :**     Mesure de la réactivité globale de l'animal dans le test de l'open field. Le XA à la dose de 37,5 mg /kg (dose non sédative) favorise le déplacement de l'animal au centre du champ ouvert éclairé par rapport aux animaux contrôles.

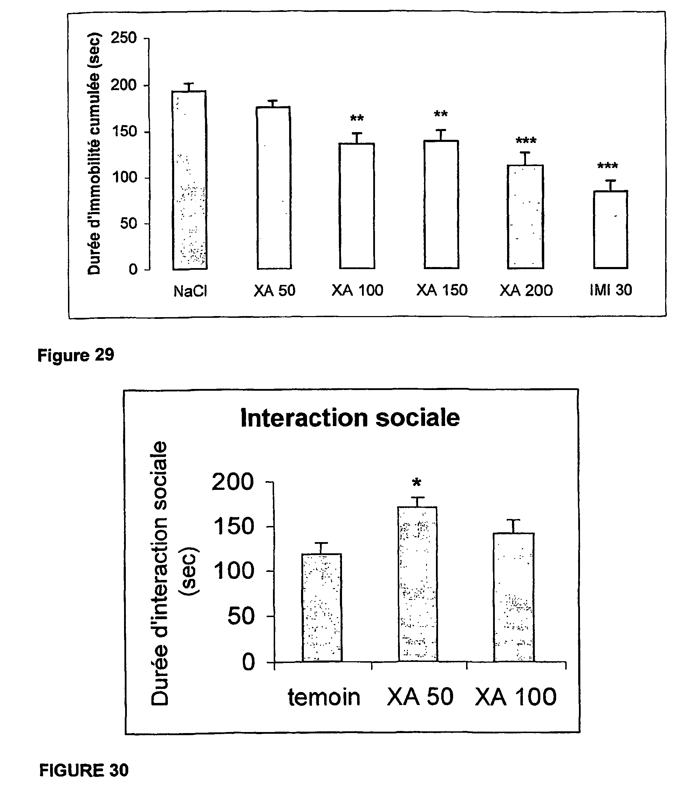

**Figure 29 :**     Effet / dose du XA dans le test de Porsolt (test de la nage). La durée d'immobilité décroit avec l'augmentation de la dose de XA. Les rats traités à l'imipramine 30 mg / kg servent de réferences positives.

**Figure 30:**     Dans l'open field, une dose de XA de 50 mg / kg induit une augmentation de la durée des interactions sociales par rapport à l'animal non traité. Ce résultat est également en faveur d'un effet anxiolytique du XA.

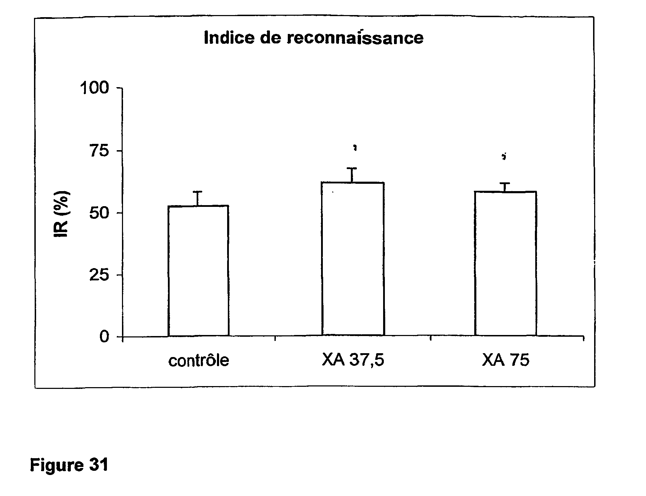

**Figure 31 :**     Effet de XA sur les processus de mémorisation dans le test de reconnaissance d'objets.

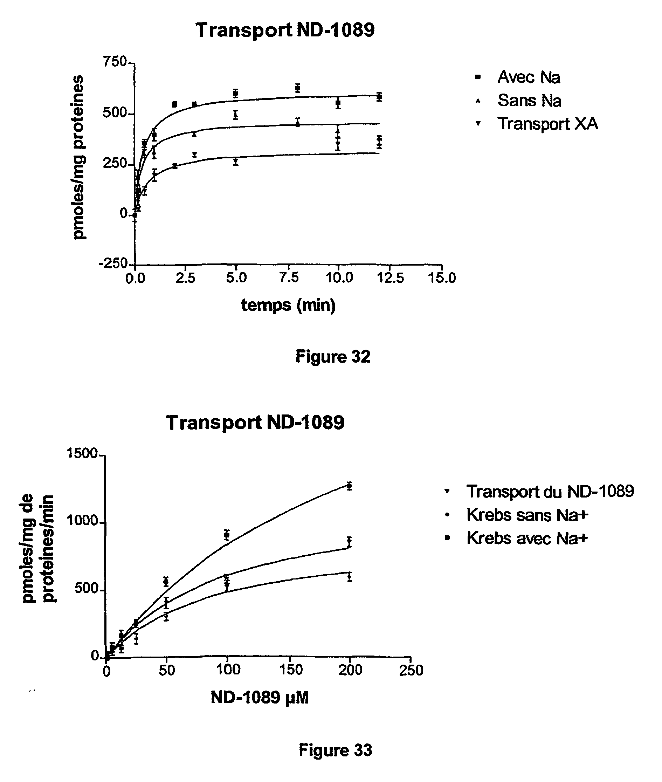

**Figure 32 :**     Cinétique du transport de XA (ND-1089) dans les cellules NCB-20. La vitesse optimum du transport est atteinte après environ 1 minute.

**Figure 33 :**     Transport de XA (ND-1089) dans les cellules NCB-20 en fonction de la concentration en [$^3$H]XA. La représentation de Scatchard permet de déterminer les paramètres mathématiques du transport intracellulaire du ND-1089 soit un Km de 105 $\mu$M et une Vmax de 1229 pmoles/mg protéine/minute.

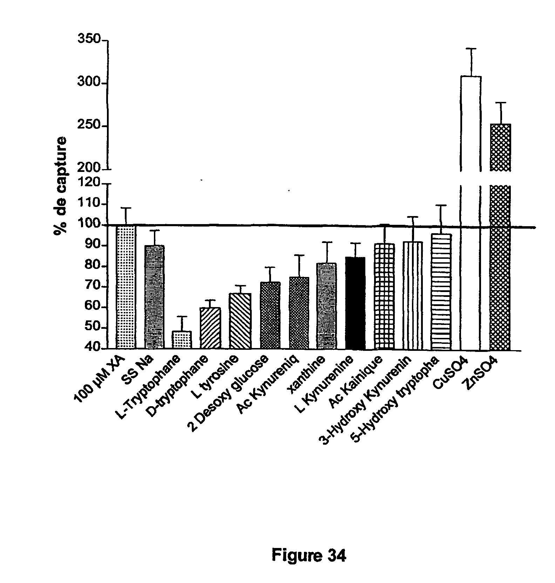

**Figure 34:**     histogramme exprimé en pourcentage de capture par référence au transport en présence de XA radioactif seul (=100 %).

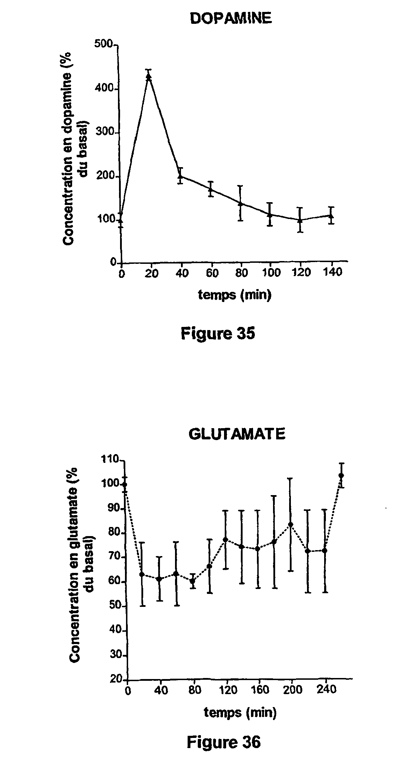

**Figure 35:** libération extracellulaire frontale après rétrodialyse de XA 20 M pendant 20 minutes exprimée en concentration de dopamine (% du basal) par rapport au temps (minutes) dans les dialysats

**Figure 36 :** libération extracellulaire frontale après rétrodialyse de XA 20 M pendant 20 minutes exprimée en concentration de glutamate (% du basal) par rapport au temps (minutes) dans les dialysats

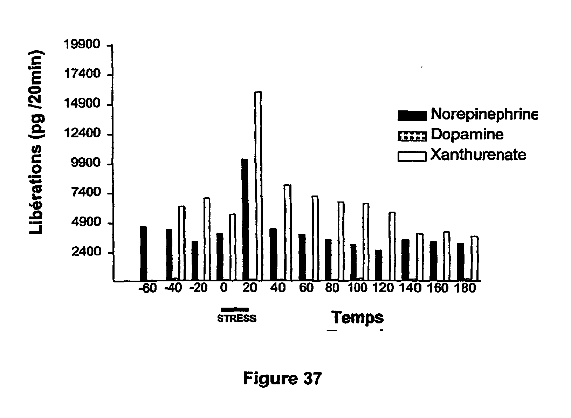

**Figure 37:** libération extracellulaire frontale après rétrodialyse de XA 20 M pendant 20 minutes exprimée en concentration de glutamate (% du basal) par rapport au temps (minutes

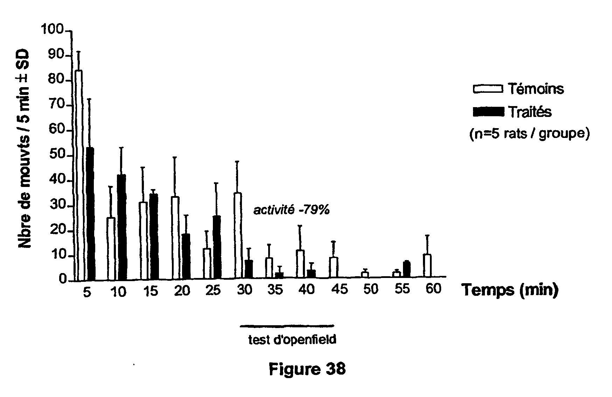

**Figure 38 :** Actographie obtenue avec administration d'un ligand se fixant sur le site allostérique pour l'acide kynurénique, partie intégrante du site récepteur de XA qui est un dérivé de l'acide xanthurénqiue, intitulé ND-7000, à 100 mg / kg per os (PO)

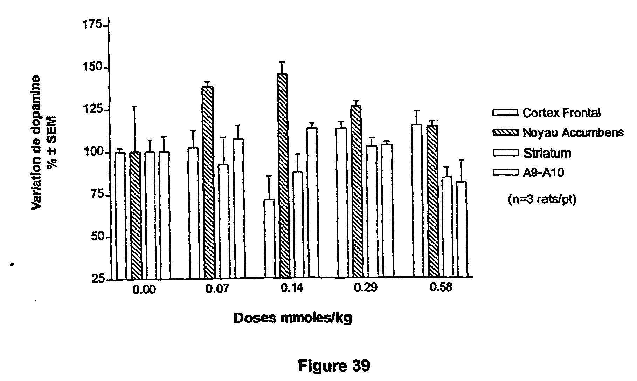

**Figure 39 :** Effet dose/réponse après administration (P.O.) d'un ligand agoniste du récepteur XA, dérivé de l'acide xanthurénique, intitulé ND-1301, sur la dopamine tissulaire dans différentes régions cérébrales

## SECTION EXPERIMENTALE

### 1. MISE EN EVIDENCE ET PROPRIETES DE LA LIBERATION EXTRACELLULAIRE D'ACIDE XANTHURENIQUE (XA) IN VIVO DANS LE CORTEX PREFRONTAL DU RAT.

En combinant la stimulation électrique de l'aire tegmentaire ventrale (A10) et la microdialyse in vivo du cortex préfrontal chez le rat, une libération extracellulaire de XA a pu être mis en évidence. Cette libération peut-être reproduite par dépolarisation locale a l'aide de fortes concentrations d'ions potassium ou de quantités micromolaires de veratridine. La libération de XA est calcium-dépendante et est bloquée par la tétrodotoxine qui empêche la dépolarisation neuronale locale. Le XA est donc libéré dans cette région du cerveau au moins, selon des caractéristiques identiques à celles des autres neurotransmetteurs connus, c'est à dire selon un mécanisme exocytotique induit par la dépolarisation neuronale.

### Matériels et Méthodes

#### • Animaux

Des rats males Wistar pesant 350 à 400 g ont été utilisés pour les expériences. Les animaux sont placés en cage individuelle de plastique et soumis à un cycle lumière/obscurité de 7 heures/ 19 heures et 19 heures/7 heures. L'accès à la nourriture et à l'eau est libre. Les interventions sur les animaux ont été menées dans la stricte application des règles de la directive européenne du 24 novembre 1986 (86/609/EEC).

#### • Procédure Chirurgicale

Les expériences ont été réalisées à l'aide de canule en I, avec une extrémité de 4 mm. La membrane de dialyse (500 $\mu$m) est en polycarbonate-polyether avec un seuil d'arrêt de 20 Kda. La membrane de dialyse est insérée dans le cortex préfrontal (CPF) tandis qu'une des extrémités de l'électrode de stimulation bipolaire est placée dans CPF et l'autre dans le VTA. L'intervention est réalisée sous anesthésie à la kétamine.

#### • Protocole de Microdialyse

Les expériences sont réalisées chez l'animal conscient, 24 à 72 heures après l'intervention chirurgicale. La composition du milieu de perfusion est la suivante : 147 mM NaCI, 1.2 mM CaCl$_2$, 1.2 mM MgCl$_2$, et 4.0 mM KCI, pH 6.5. Dans le cas des stimulations à fortes concentrations de potassium, les ions sodium sont diminués de la même valeur. Dans le cas des stimulations en l'absence d'ions calcium, 2.0 mM EGTA sont rajoutés dans le milieu de dialyse. Les dialysats sont collectés à une vitesse de 2 $\mu$l/min, toutes les 5 minutes et sont immédiatement stockés dans l'azote liquide jusqu'à l'analyse.

#### • Analyse de la dopamine et de l'acide xanthurénique

Ces deux composés sont quantifiés par chromatographie HPLC avec détection électrochimique. Le système chromatographique consiste en une colonne 25 cm X 4.6 mm Hypersyl C18, maintenue à température constante. La phase mobile est un tampon 0.05 M NaH$_2$PO$_4$ + 0.1 mM EDTA et 6% méthanol. L'identification des pics s'effectue par comparaison avec les temps de rétention mesurés avec des solutions standards de calibration.

**• Protocole de stimulation électrique**

Le VTA a été stimulé pendant une période de 10 minutes, avec des trains de 30 impulsions de durée 0.5 msec pendant 100 millisec, d'intensité 100 ou de 200 μA et à une fréquence de 25 Hz.

**• Détermination des rendements in vitro**

Ces déterminations sont destinées à estimer le passage de la dopamine et de XA au travers de la membrane de dialyse pendant les expériences in vivo. Les rendements de dialyse in vitro ont respectivement été d'environ 28% et d'environ 15% pour respectivement XA et la dopamine pour une vitesse de dialyse de 1 μl/min à température ambiante.

**Résultats**

**• Détermination des concentrations de XA dans le milieu extracellulaire du CPF**

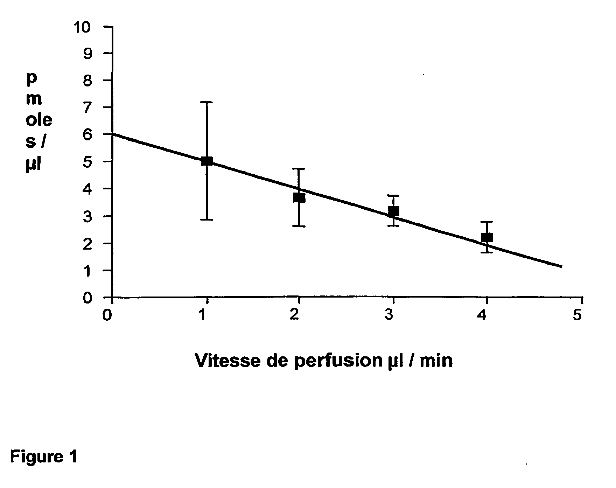

Ces études ont été réalisées en déterminant les taux variables de XA dialysés à différents rendements de dialyse. Les divers rendements de dialyse ont été modifiés par le changement graduel de la vitesse du liquide de dialyse (respectivement 1,2,3 et 4 μl / minute) et détermination à chaque fois de la concentration de XA. Par extrapolation à vitesse zéro, on a un rendement de 100 % et une valeur de la concentration de XA dans le liquide de dialyse égal à sa concentration dans le milieu extracellulaire. L'expérience, menée sur 3 différents rats, a donné une valeur de XA = 6.1 pmol/μl de liquide de dialyse ou de milieu extracellulaire cérébral. Ce qui donne une valeur moyenne de 6.1 μM de XA dans le milieu extracellulaire du CPF (figure 1).

**• Libération de XA dans le CPF après stimulation électrique.**

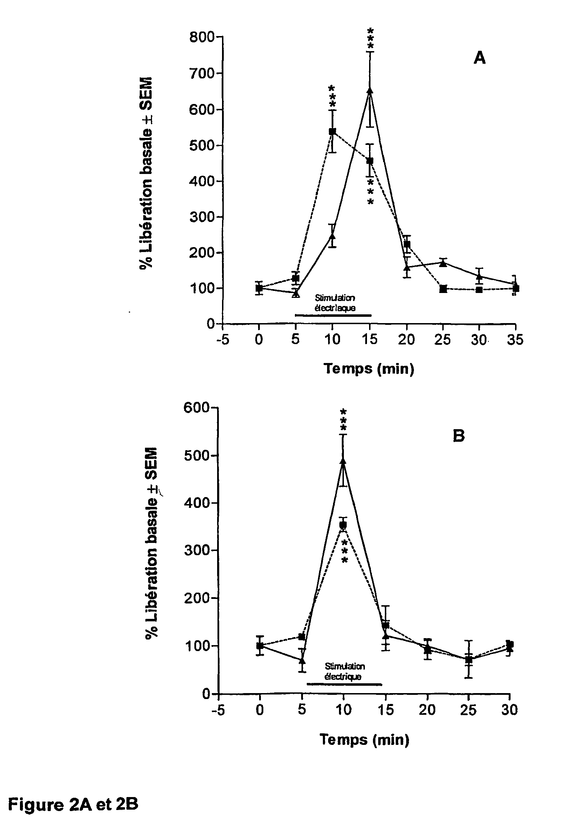

Dans deux séries d'expériences, les stimulations électriques ont été de 100 ou 200 pA, pendant 10 minutes. Après insertion de la sonde et une heure de dialyse, la ligne de base à été établie par dialyse durant la deuxième heure (16 à 18 pmol/5 min). Une stimulation à 100 ou 200 μA à donné le même résultat, indiquant que la totalité du compartiment libérable dans nos conditions, avait été libéré. Par rapport à une ligne de base arbitrairement fixée à 100%, la libération électriquement induite de XA atteint 539% alors que la libération de dopamine atteint 655%. La libération s'installe dés la stimulation, atteint un maximum après environ 5 minutes de stimulation, puis revient à la ligne de base, même si la stimulation se poursuit. Une cinétique plus précise ne peu être décrite, compte tenu des conditions de dialyse (prélèvements toutes les 5 minutes). Les résultats sont présentés sur la figure 2.

**• Libération de XA dans le CPF après dépolarisation locale par de fortes concentrations d'ions potassium ou par 50 μM de veratrine.**

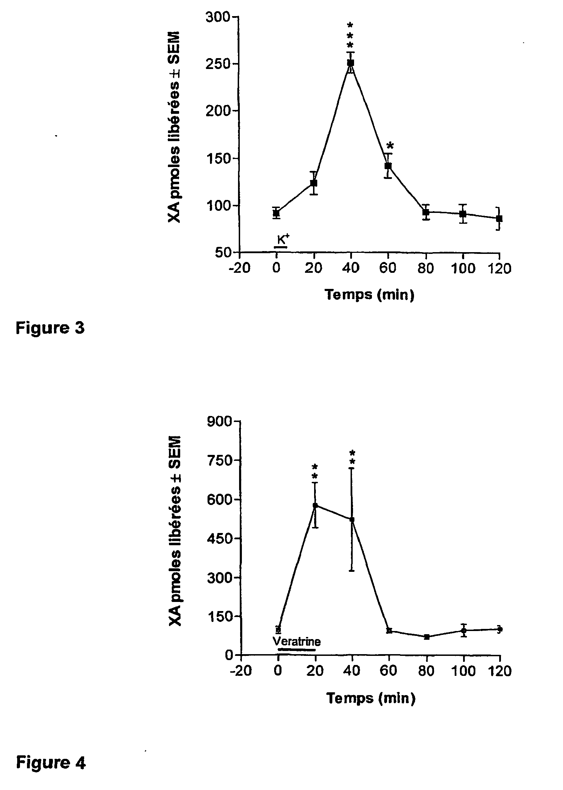

La libération de XA dans l'espace extracellulaire du CPF à été également testée après dépolarisation locale par de fortes concentrations d'ions potassium placés dans la sonde de dialyse (100 mM de KCl pendant 5 minutes). Avant stimulation, la libération de base était d'environ 92 pmol / 20 min. Après la dépolarisation, la libération de XA augmente progressivement pour atteindre 250% du niveau de base après 40 minutes. Puis, le niveau de base se rétablit progressivement et rejoint les niveaux antérieurs 80 minutes après la dépolarisation (figure 3).
Dans le cas de la veratrine, celle-ci a été placée dans la sonde à la concentration de 50 μM pendant 20 minutes. Le niveau XA de base (avant la dépolarisation) était d'environ 96 pmol / 20 minutes. Après application de la veratrine, un pic de XA est rapidement libéré, atteignant environ 600% du niveau de base au bout de 20 minutes, puis le niveau de base antérieur est progressivement rétabli en 60 minutes environ (figure 4).

**• La libération de XA induite dans le CPF par la stimulation électrique est un phénomène calcium-dépendent**

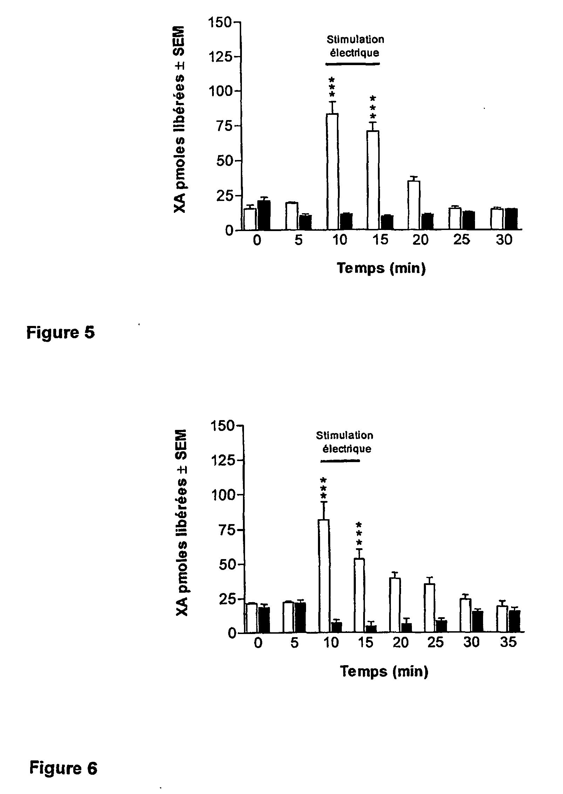

Dans ces études, un groupe de rat est soumis à une stimulation électrique dans le VTA et on mesure la libération de XA dans le cortex frontal. La ligne de base est à 19 pmol/5 min et après stimulation électrique, la libération est à 70-75 pmol de XA pour 5 min de dialyse. La même expérience est refaite, mais dans ce cas le milieu de dialyse ne contient pas d'ions calcium, mais 2.0 mM EGTA. Dans ces conditions, la libération de base de XA est de 10 pmol / 5 min et la stimulation électrique ne produit aucun accroissement de la libération de XA (figure 5).

**• La libération de XA dans le CPF après stimulation électrique est inhibée par la tétrodotoxine, bloqueur des canaux sodiques sodium-dépendant.**

L'étude est réalisée sur des rats témoins chez lesquels on mesure la libération de XA dans le CPF après stimulation électrique. Les mêmes expériences sont ensuite répétées, mais en plaçant 2.0 de tétrodotoxine dans la sonde de dialyse pendant 10 minutes. Dans ces dernières conditions, la libération de XA induite par la stimulation électrique est complètement bloquée (figure 6).

## 2. DISTRIBUTION HETEROGENE DE XA DANS LE CERVEAU DE RAT APRES ADMINISTRATION PERIPHERIQUE.

Méthodes: Les rats sont injectés par voie i.p. avec 50 mg / kg de XA puis sacrifiés 30 minutes après par irradiation aux micro-ondes. Le cerveau est disséqué et le XA est dosé dans 20 régions différentes du cerveau.

**Résultats :** Les résultats sont présentés dans le tableau ci-dessous (ng / g de cerveau).

| Structures cérébrales | Concentrations physiologiques | Après injection | Augmentation |
|---|---|---|---|
| Cortex préfrontal | $146 \pm 90$ | $1297 \pm 351$ | 9 fois (**) |
| Cortex frontal | < 50 | $1448 \pm 165$ | 30 (***) |
| Cortex pariétal | $416 \pm 23$ | $2565 \pm 692$ | 6 (*) |
| Cortex temporal | $110 \pm 60$ | $995 \pm 360$ | 9 (*) |
| Caudé-Putamen | $48 \pm 4$ | $595 \pm 294$ | 12 (*) |
| Amygdales | $297 \pm 13$ | $1204 \pm 180$ | 4 (*) |
| Substance noire, VTA | $397 \pm 73$ | $1307 \pm 351$ | 3 (*) |

L'accumulation de XA est surtout importante dans le cortex frontal et préfrontal (CPF), dans le cortex temporal et pariétal, dans les amygdales, les noyaux dopaminergiques et dans le striatum.

Parmi les structures cérébrales étudiées, les régions ne présentant pas d'accumulation significative d'acide xanthurénique sont les suivantes : Cortex occipital, cortex cingulaire, cortex entorhinal, cortex rétroslénial, bulbes olfactifs, pons, hippocampe, médulla oblongata, cervelet, thalamus, noyau accumbens, septum, globus pallidus et hypothalamus.

## 3. CONCENTRATIONS PHYSIOLOGIQUES DE XA DANS DIVERSES REGIONS DU CERVEAU DE RAT.

**Méthodes :** Les animaux sont sacrifiés par irradiation aux micro-ondes, les cerveaux sont rapidement disséqués sur une plaque de verre. De nombreuses structures cérébrales et divers noyaux sont isolés et conservés dans l'azote liquide jusqu'à l'analyse. Après pesée de chaque structure, les tissus sont homogénéisés dans 10 volumes d'acide perchlorique (w/v) et centrifugés. Les surnageants sont analysés par HPLC avec détection électrochimique. La limite de détection est de 0.05 nmoles /g de poids frais.

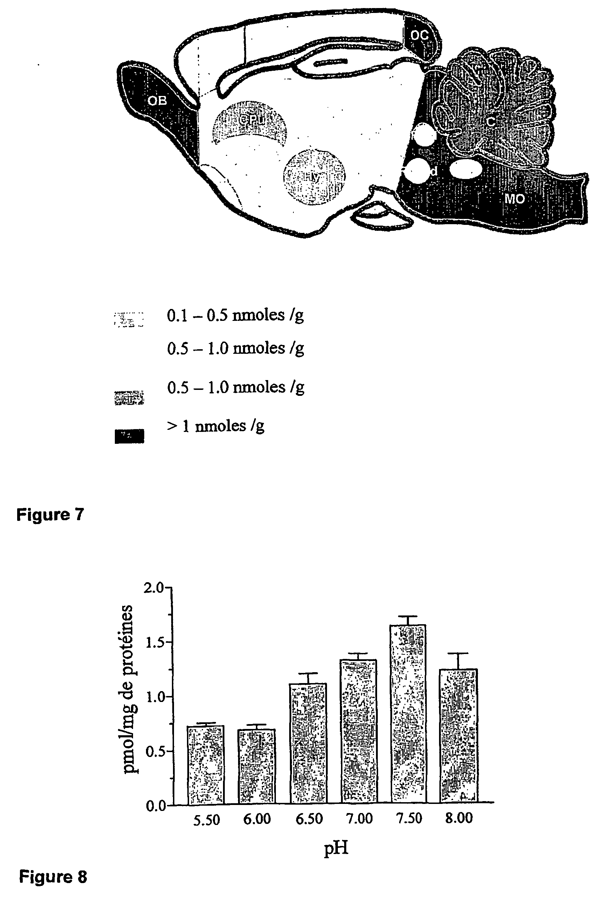

**Résultats :** Les résultats sont présentés sur la Figure 7. Ils montrent une concentration hétérogène, particulièrement élevée dans le cervelet et le bulbe olfactif.

## 4. IDENTIFICATION ET CARACTERISATION DES SITES DE FIXATION POUR L'ACIDE XANTHURENIQUE DANS LE CERVEAU DE RAT.

**Protocole de préparation des membranes synaptiques :**

Les membranes synaptiques ont été préparées selon le protocole décrit ci-après.

1°) Prélèvement rapide de deux cerveaux entiers de rats mâles Wistar (décapitation) et pesée des cerveaux.

2°) Homogénéisation des cerveaux dans 10 x le poids en volume de solution S :

S = sucrose 0.32 M

$KH_2PO_4$ 10 mM pH 6.0

EDTA 1 mM

3°) Centrifugation à 915 g à 4°C pendant 10 minutes (Du Pont Instruments, Sorvall RC-5B) et récupération du surnageant.

4°) Centrifugation à 18200 g à 4°C pendant 20 minutes (Du Pont Instruments, Sorvall RC-5B).

5°) Prélèvement du culot et éclatement des synaptosomes dans 70 x le poids en volume d'eau distillée à 0°C. Polytroner 30 secondes à vitesse maximale.

6°) Partage dans des tubes de centrifugation Beckman selon le volume et centrifugation à 51000 g à 4°C (Beckman, ultracentrifugeuse L8-70M) pendant 20 minutes.

7°) Lavage des culots dans le tampon $KH_2PO_4$ 50 mM pH 6.0 à 0°C

8°) Partage dans des tubes de centrifugation Beckman selon le volume et centrifugation à 51000 g à 4°C (Beckman, ultracentrifugeuse L8-70M) pendant 20 minutes.

9°) Récupération et congélation des culots à -80°C.

Partie 1 : ETUDE DES CARACTERISTIQUES PHARMACOLOGIQUES DU SITE DE LIAISON DE L'ACIDE XANTHURENIQUE SANS IONS

**Protocole de liaison**

Protocole classique :

Le binding est réalisé dans du tampon Pipes 50 mM pH 7,4 à 0°C (sur glace) en présence de membranes synaptiques (quantité protéique variant de 0.1 à 0.3 mg par tube), d'acide xanthurénique tritié ([3H]-AX en concentration variable selon le type d'expérience) et soit du tampon (pour la détermination de la liaison totale: LT) soit de l'acide xanthurénique "froid" non tritié (Sigma) à 2 mM (pour la détermination de la liaison non spécifique: LNS). La soustraction entre liaison totale et liaison non spécifique donne la liaison spécifique: LS. Le temps d'incubation est de 25 min. La filtration qui permet la séparation du [3H]-AX libre du [3H]-AX fixé à son (ses) site (s) de binding s'effectue par aspiration rapide du milieu d'incubation à travers des filtres en fibres de verre (GF/B) whatman qui sont ensuite lavés de façon successive 2 fois par du tampon Pipes 50 mM pH 7,4 froid (en tout 3x3 ml). Les filtres sont placés dans des fioles de comptage et on y ajoute 5 ml de Rotiszint® (Roth). Le comptage s'effectue dans un compteur à scintillation liquide (Beckman LS6000sc).

Protocole pour la détermination de l'effet pH :

Le binding est réalisé selon le modèle classique: Le milieu d'incubation est préparé à partir de tampon Pipes 50 mM à différents pH (pH étudiés : 5,5 ; 6,0 ; 6,5 ; 7,0 ; 7,5 ; 8,0). La concentration en acide xanthurénique tritié ([3H]-AX) est de 200 nM et pour l'étude de la liaison non spécifique on utilise une concentration de 2 mM d'acide xanthurénique non tritié (AX). La soustraction entre liaison totale et liaison non spécifique donne la liaison spécifique qui varie en fonction du pH. Les résultats présentés dans la Figure 8 montrent que le pH optimum de binding de l'acide xanthurénique est de 7,4-7,5.

Mesure de la zone de proportionnalité en fonction des protéines

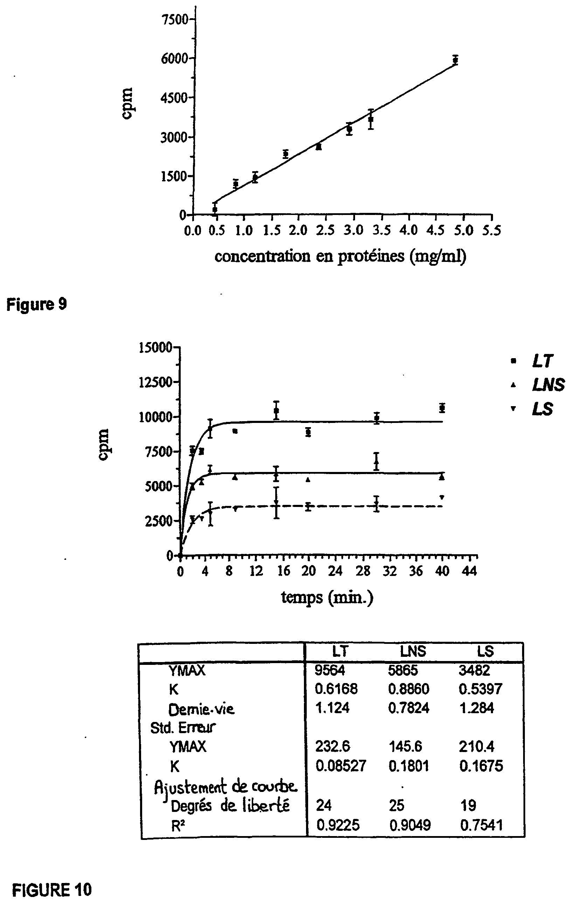

Le binding est réalisé selon le modèle classique: Le milieu d'incubation est préparé à partir de tampon Pipes 50 mM pH 7,4 (pH optimum). On fait varier la quantité protéique (0.04 mg à 0.5 mg par tube). La concentration en acide xanthurénique tritié ([3H]-AX) est de 200 nM et pour l'étude de la liaison non spécifique on utilise une concentration de 2 mM d'acide xanthurénique non tritié (AX). La soustraction entre liaison totale et liaison non spécifique donne la liaison spécifique qui varie en fonction de la quantité protéique : jusqu'à une quantité protéique de 0.5 mg par tube on reste dans la zone de proportionnalité. Les résultats sont présentés sur la Figure 9. Pour les expériences ultérieures, on travaillera entre 0.15 à 0.3 mg de protéines par tube.

Mesure des constantes cinétique du binding

** Constante du taux d'association ou ($k_1$ ou kon) :

Le binding est réalisé selon le modèle classique: Le milieu d'incubation est préparé à partir de tampon Pipes 50 mM pH 7,4 (pH optimum). La concentration en acide xanthurénique tritié ([3H]-AX) est de 200 nM et pour l'étude de la liaison non spécifique on utilise une concentration de 2 mM d'acide xanthurénique non tritié (AX). La préparation membranaire synaptique est ajoutée dans chaque tube et on procède à la filtration rapide aux différents temps étudiés; les temps d'incubation varient de 1 minute à 40 minutes. La soustraction entre liaison totale et liaison non spécifique donne la liaison spécifique qui augmente en fonction du temps pour atteindre un équilibre : l'équilibre est atteint aux alentours de 10-15 minutes. L'analyse au logiciel Graphpad Prism de l'équation de l'association exponentielle donne une constante du taux observé $K_{ob}$ exprimé en min$^{-1}$ qui n'est pas le même que le kon. La valeur du kob est de 0.53 $\pm$ 0.16 min$^{-1}$ (Figure 10).

Equation utilisée pour le calcul du kon :

$$kon = \frac{kob - koff}{[radioligand]} \quad en \ M^{-1} \ min^{-1}$$

**\*\* Constante du taux de dissociation ou ($k_{-1}$ ou koff) :**

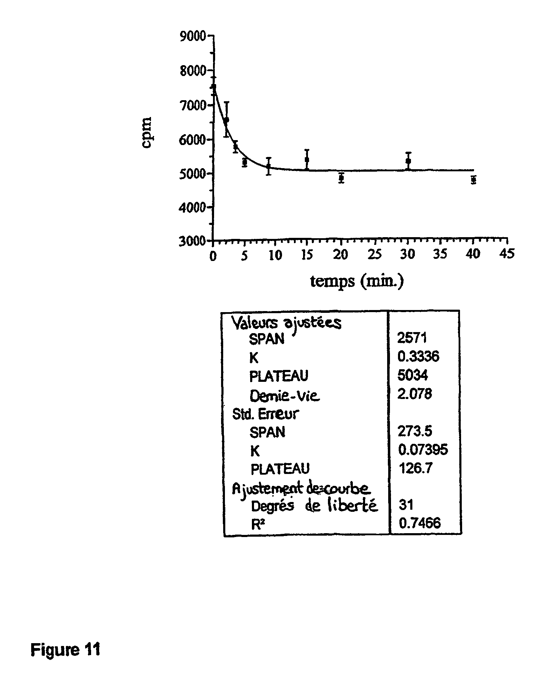

Le binding est réalisé selon le modèle classique: Le milieu d'incubation est préparé à partir de tampon Pipes 50 mM pH 7,4 (pH optimum). On procède à une incubation de la préparation membranaire synaptique avec l'acide xanthurénique tritié (200 nM) pendant 25 minutes (liaison totale) puis on y ajoute l'acide xanthurénique non radiomarqué (2 mM) que l'on laisse incuber pendant différents temps pour observer la dissociation et on procède à la filtration rapide; les temps d'incubation varient de 1 minute à 45 minutes. Initialement le [³H]-AX est lié à son site de binding, l'équilibre est atteint puis plus on incube pendant des temps prolongés avec l'AX non radiomarqué plus il y a une diminution du binding qui traduit la dissociation du complexe site de binding-[³H]-AX. La dissociation est rapide et l'analyse au logiciel Graphpad Prism de l'équation de la dissociation exponentielle donne la constante du taux de dissociation koff exprimé en min$^{-1}$. La valeur du koff est de 0.33 $\pm$ 0.07 min$^{-1}$ (Figure 11).
\*\*Ces deux constantes permettent d'extrapoler de façon approximative une valeur pour le $K_d$ = koff/kon = 330 nM.

<u>Expérience de saturation, mesure réelle du Kd et Bmax</u>

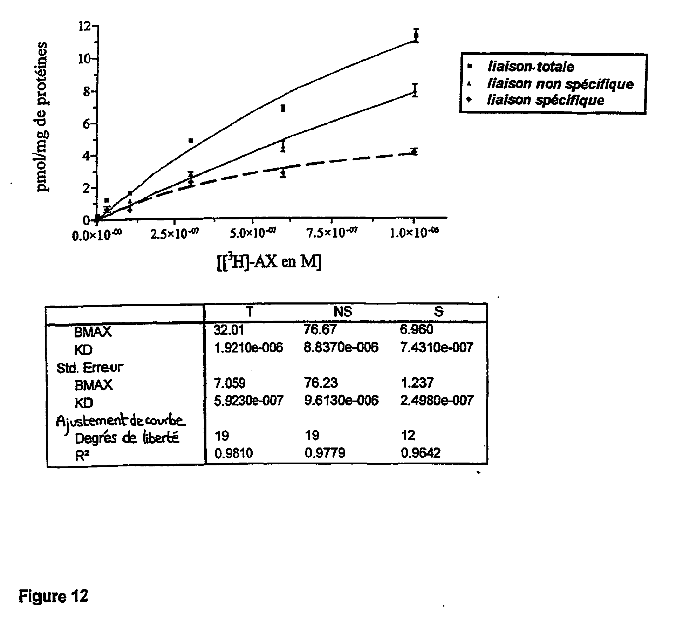

Le binding est réalisé dans du tampon Pipes 50 mM pH 7,4 à 0°C (sur glace) en présence de membranes synaptiques (quantité protéique variant de 0.1 à 0.3 mg par tube), d'acide xanthurénique tritié et soit du tampon (pour la détermination de la liaison totale) soit de l'acide xanthurénique non radiomarqué à 2 mM (pour la détermination de la liaison non spécifique). Le temps d'incubation est de 25 min. puis on procède à la filtration.
On fait varier la concentration en acide xanthurénique tritié de façon croissante afin d'arriver à une occupation des sites de liaison maximale (plateau de saturation). On réalise la même chose en présence d'un excès d'acide xanthurénique non radiomarqué afin de déterminer la liaison non spécifique du [³H]-AX. Ainsi en soustrayant la liaison totale de la liaison non spécifique on obtient la liaison spécifique de l'acide xanthurénique à son (ou ses) site(s) de binding. L'analyse au logiciel Graphpad Prism permet de déterminer l'affinité de l'acide xanthurénique pour son (ou ses) site(s) de liaison : le $K_d$ est de 743 nM $\pm$ 250 nM. De même le logiciel Graphpad Prism permet de déterminer le nombre de sites de liaison présents sur une préparation de membranes d'origine synaptique obtenues à partir de cerveaux entiers de rat : le Bmax est de 6.9 pmol/mg de protéines $\pm$ 1.2 pmol/mg de protéines (Figure 12).

<u>Expérience de compétition, mesure d'une C150 (concentration inhibitrice 50)</u>

Le binding est réalisé dans du tampon Pipes 50 mM pH 7,4 à 0°C (dans la glace) en présence de membranes synaptiques (quantité protéique variant de 0.1 à 0.3 mg par tube), d'acide xanthurénique tritié (200 nM) et soit du tampon (pour la détermination de la liaison totale), soit de l'acide xanthurénique non radiomarqué à 2 mM (pour la détermination de la liaison non spécifique) et soit des concentrations variables d'une molécule non radiomarquée. Si la molécule reconnaît le site de liaison du [³H]-AX de façon réversible alors les 2 ligands vont entrer en compétition et on obtiendra une courbe de déplacement du [³H]-AX en fonction de la concentration de la molécule compétitrice. Le temps d'incubation est de 20-25 min. puis on procède à la filtration.
Le radioligand [³H]-AX a été préparé par hydrogénation catalytique de l'acide 5,7-dichloro-8-hydroxyquinoléine-2-carboxylique en présence de palladium sur charbon (10%) dans le méthanol et en présence de tritium gaz.
Le composé, après purification par HPLC, présente une activité de 10Ci/mmole.
L'analyse au logiciel Graphpad Prism permet de déterminer la CI50 qui représente la concentration de la molécule qui bloque 50 % du binding de l'acide xanthurénique. Lorsque l'on effectue une expérience de compétition entre le [³H]-AX et l'AX non radiomarqué, on obtient une courbe de déplacement dont l'analyse au logiciel Graphpad Prism nous donne un modèle de liaison à deux sites, une CI50 à 300 nM et une CI50 à 57 $\mu$M. Selon ce protocole, on peut effectuer des expériences de compétition avec des molécules de synthèse et ainsi identifier ou caractériser des agonistes ou antagonistes, en particulier ayant une meilleure affinité pour le récepteur de l'acide xanthurénique que l'AX lui-même.

Pour cela avant de procéder à une expérience de compétition classique pour déterminer une CI50, on effectue d'abord un "screening" c'est à dire que l'on teste à une concentration relativement basse (10 $\mu$M) les molécules sur le déplacement de 200 nM de [3H]-AX (en gardant comme contrôle l'AX non radiomarqué) et l'on regarde si cette concentration déplace de façon identique ou meilleure cette même concentration en AX non radiomarqué. Si oui, on détermine la CI 50.

L'étude du déplacement du [3H]-AX par des métabolites du tryptophane (L-kynurénine, 3OH-DL-kynurenine, 5 hydroxy-L- tryptophane, acide picolinique , acide 3 hydroxy anthranilique) dont l'acide xanthurénique fait partie a également été réalisée en screening à 200 $\mu$M. Aucun des composés ne déplace de façon significative l'acide xanthurénique. Par contre le screening réalisé sur l'acide kynurénique révèle qu'il n'y a pas de déplacement du [3H]-AX de son (ses) site(s) de liaison mais une potentialisation de cette liaison. En effet on obtient une liaison de l'acide xanthurénique tritié supérieure à ce que l'on obtient dans les conditions normales de binding.

Effet des ions sur la liaison de l'acide xanthurénique à son (ses) sites de liaison

L'étude de l'influence d'ions sur la liaison de l'acide xanthurénique à son (ses) site(s) de liaison est réalisé dans un tampon Tris Maléate 50 mM pH 6.5 à 0°C (dans la glace) en présence de membranes synaptiques (quantité protéique variant de 0.1 à 0.3 mg par tube), d'acide xanthurénique tritié (200 nM) et soit du tampon (pour la détermination de la liaison totale), soit de l'acide xanthurénique non radiomarqué à 2 mM (pour la détermination de la liaison non spécifique) et une concentration de 1 mM en ions. Le temps d'incubation est de 25 minutes. Les ions suivants ont été testés : $Cu^{2+}$ ($CuCl_2$); $Zn^{2+}$ ($ZnCl_2$); $Mg^{2+}$ ($MgCl_2$); $Mn^{2+}$ ($MnCl_2$); $Cd^{2+}$ ($CdCl_2$); Sn2+ (SnCl2), $Fe^{3+}$ ($FeCl_3$).

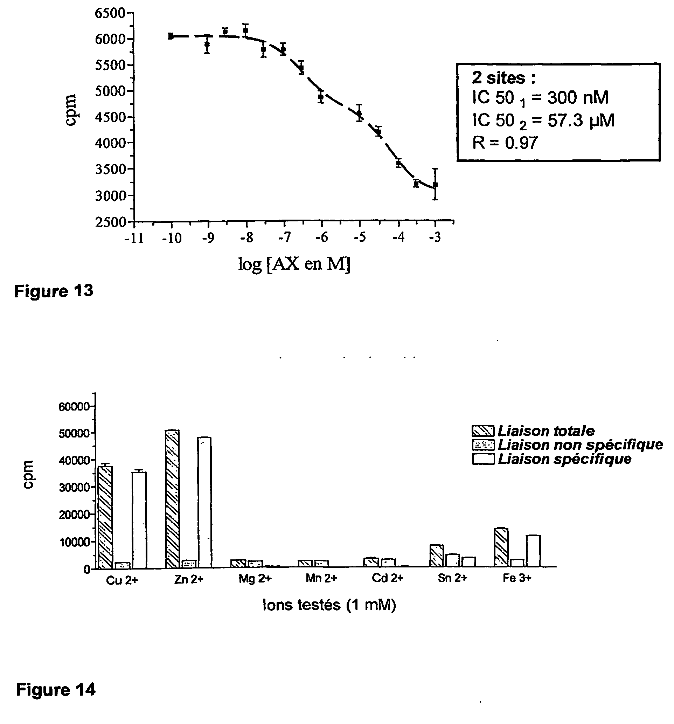

Les courbes $EC_{50}$ pour les différents ions (Figure 14) donnent la préférence aux ions cuivriques : ceux-ci possèdent un $EC_{50}$ d'environ 100 $\mu$M alors que les ions zinc possèdent un $EC_{50}$ d'environ 500 $\mu$M. Une concentration de 100-200 $\mu$M en ions cuivriques représente approximativement les concentrations endogènes cérébrales.

D'autre part, au cours de nos recherches sur l'existence de ligands endogènes cérébraux pouvant jouer un rôle au niveau du site de binding XA ou au niveau d'un site modulateur de ce récepteur, nous avons constaté que l'adénine et ses dérivés [adénosine, adénosine diphosphate (ADP) ou adénosine triphosphate (ATP)] présentaient une affinité significative pour le site xanthurénique et inhibaient la fixation de XA tritié. Il est très probable que ces dérivés jouent un rôle important dans la modulation de l'activité du site XA au niveau cérébral. Ces molécules peuvent représenter des modèles pour la réalisation de ligands analogues structuraux pouvant interférer avec les sites XA pour la confection d'outils thérapeutiques ou pharmacologiques.

**Partie 2 : ETUDE DES CARACTERISTIQUES PHARMACOLOGIQUES DU SITE DE LIAISON DE L'ACIDE XANTHURENIQUE EN PRESENCE D'IONS CUIVRE (Cu2+)**

Protocole de Binding classique (protocole établi à partir des études du pH, de la proportionnalité des constantes cinétique et de la saturation)

Le binding est réalisé dans du tampon Tris Maléate 50 mM pH 6.5 à température ambiante en présence de membranes synaptiques (quantité protéique variant de 0.1 à 0.25 mg par tube), d'acide xanthurénique tritié ([3H]-AX en concentration variable selon le type d'expérience), 200 $\mu$M de $CuCl_2$ et soit du tampon (pour la détermination de la liaison totale: L*T*) soit de l'acide xanthurénique "froid" non tritié (Sigma) à 2 mM (pour la détermination de la liaison non spécifique: L*NS*). La soustraction entre liaison totale et liaison non spécifique donne la liaison spécifique: LS. Le temps d'incubation est de 25 min. La filtration qui permet la séparation du [3H]-AX libre du [3H]-AX fixé à son (ses) site (s) de binding s'effectue par aspiration rapide du milieu d'incubation à travers des filtres en fibres de verre (GF/B) Whatman qui sont ensuite lavés de façon successive 2 fois par du tampon Tris Maléate 50 mM pH 6.5 à température ambiante (en tout 3x3 ml). Les filtres sont placés dans des fioles de comptage et on y ajoute 5 ml de Rotiszint® (Roth). Le comptage s'effectue dans un compteur à scintillation liquide (Beckman LS6000sc).

Effet pH :

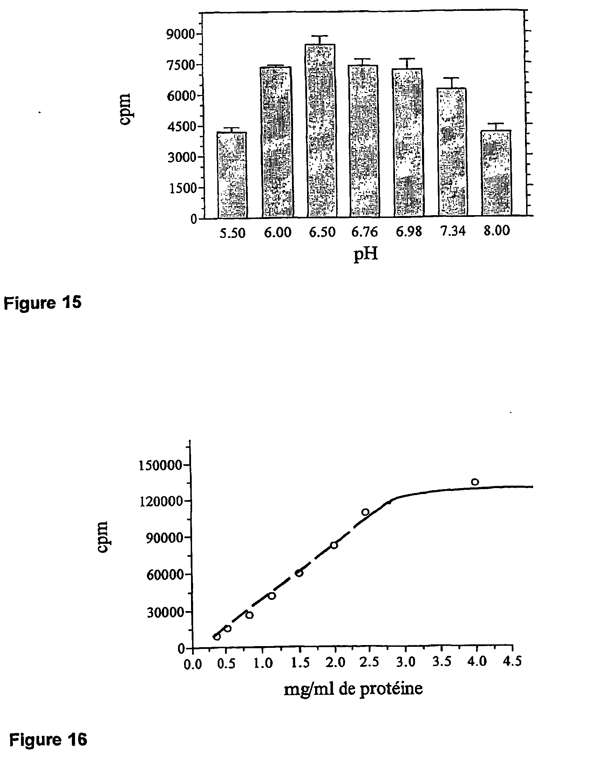

Le binding est réalisé selon le modèle classique: Le milieu d'incubation est préparé à partir de tampon Tris Maléate 50 mM à température ambiante à différents pH (pH étudiés : 5,5 ; 6,0 ; 6,5 ; 7,0 ; 7,5 ; 8,0). La concentration en acide xanthurénique tritié ([3H]-AX) est de 200 nM (+ 20 $\mu$M d'acide xanthurénique) et pour l'étude de la liaison non spécifique on utilise une concentration de 2 mM d'acide xanthurénique non tritié (AX). Le milieu d'incubation contient 200 $\mu$M de $CuCl_2$. La soustraction entre liaison totale et liaison non spécifique donne la liaison spécifique qui varie en fonction du pH (Figure 15) : le pH optimum de binding de l'acide xanthurénique est de 6,4-6,5.

Mesure de la zone de proportionnalité en fonction des protéines :

Le binding est réalisé selon le modèle classique: Le milieu d'incubation est préparé à partir de tampon Tris Maléate 50 mM pH 6.5 à température ambiante (pH optimum). On fait varier la quantité protéique (0.07 mg à 0.4 mg par tube). La concentration en acide xanthurénique tritié ([$^3$H]-AX) est de 200 nM et pour l'étude de la liaison non spécifique on utilise une concentration de 2 mM d'acide xanthurénique non tritié (AX). Le milieu d'incubation contient 200 $\mu$M de $CuCl_2$. La soustraction entre liaison totale et liaison non spécifique donne la liaison spécifique qui varie en fonction de la quantité protéique : jusqu'à une quantité protéique de 0.25 mg par tube on reste dans la zone de proportionnalité (Figure 16). Pour les expériences ultérieures, on travaillera entre 0.10 à 0.25 mg de protéines par tube.

Mesure de l'effet dose du cuivre sur le binding de l'acide xanthurénique

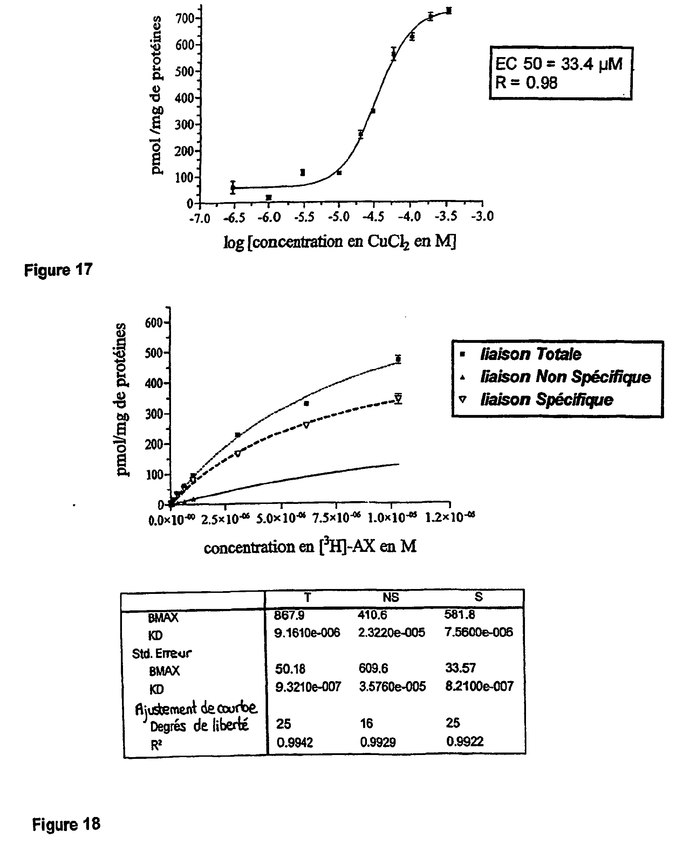

Le binding est réalisé selon le modèle classique: Le milieu d'incubation est préparé à partir de tampon Tris Maléate 50 mM pH 6.5 à température ambiante (pH optimum). On fait varier la concentration en cuivre ($CuCl_2$) de $3.10^{-7}$ M à $3.10^{-4}$ M. La concentration en acide xanthurénique tritié ([$^3$H]-AX) est de 400 nM (et 20 $\mu$M d'acide xanthurénique) et pour l'étude de la liaison non spécifique on utilise une concentration de 2 mM d'acide xanthurénique non tritié (AX). On obtient une courbe effet dose dont l'analyse au logiciel Graphpad Prism nous donne une EC50 (concentration efficace 50) à 33.4 $\mu$M avec un coefficient de Hill de 1.8 (Figure 17).

Expérience de saturation, mesure réelle du $K_d$ et $B_{max}$

Le binding est réalisé dans du tampon Tris Maléate 50 mM pH 6.5 à température ambiante en présence de membranes synaptiques (quantité protéique variant de 0.10 à 0.25 mg par tube), d'acide xanthurénique tritié et soit du tampon (pour la détermination de la liaison totale) soit de l'acide xanthurénique non radiomarqué à 2 mM (pour la détermination de la liaison non spécifique). Le temps d'incubation est de 25 min. puis on procède à la filtration.
On fait varier la concentration en acide xanthurénique tritié de façon croissante afin d'arriver à une occupation des sites de liaison maximale (plateau de saturation). On réalise la même chose en présence d'un excès d'acide xanthurénique non radiomarqué afin de déterminer la liaison non spécifique du [$^3$H]-AX. Ainsi en soustrayant la liaison totale de la liaison non spécifique on obtient la liaison spécifique de l'acide xanthurénique à son (ou ses) site(s) de binding. L'analyse au logiciel Graphpad Prism permet de déterminer l'affinité de l'acide xanthurénique pour son (ou ses) site(s) de liaison : le $K_d$ est de 7.56 $\mu$M $\pm$ 0.8 $\mu$M. De même le logiciel Graphpad Prism permet de déterminer le nombre de sites de liaison présents sur une préparation de membranes d'origine synaptique obtenues à partir de cerveaux entiers de rat : le Bmax est de 581.8 pmol/mg de protéines $\pm$ 33 pmol/mg de protéines (Figure 18).

Expérience de compétition, mesure d'une CI50 (concentration inhibitrice 50)

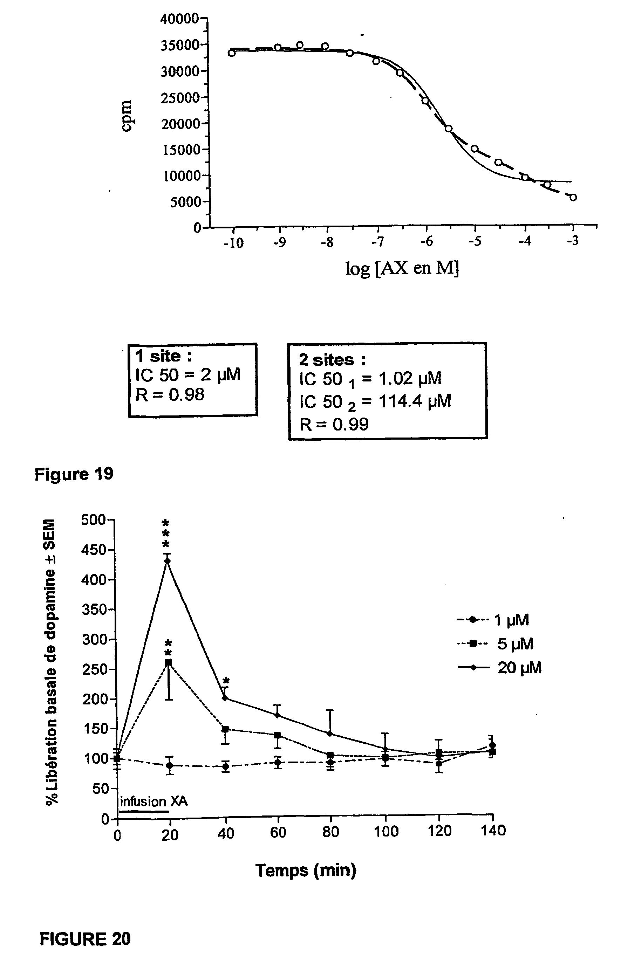

Le binding est réalisé dans du tampon Tris Maléate 50 mM pH 6.5 à température ambiante en présence de membranes synaptiques (quantité protéique variant de 0.1 à 0.3 mg par tube), d'acide xanthurénique tritié (200 nM) et soit du tampon (pour la détermination de la liaison totale), soit des concentrations variables d'AX non radiomarqué ($10^{-3}$ M à $10^{-10}$ M).
L'analyse au logiciel Graphpad Prism permet de déterminer la CI50 qui représente la concentration de la molécule qui bloque 50 % du binding de l'acide xanthurénique. L'expérience de compétition entre le [$^3$H]-AX et l'AX non radiomarqué, permet d'obtenir une courbe de déplacement dont l'analyse au logiciel Graphpad Prism nous donne un modèle de liaison à deux sites, une C150 à 1 $\mu$M et une CI50 à 114 $\mu$M (Figure 19).

**• Etude de la distribution régionale des sites de liaison de XA dans le cerveau de rat par autoradiographie quantitative suivie d'analyse d'images.**

*Méthode :* trois cerveaux de rat males Wistar adultes sont rapidement extraits après décapitation de l'animal et congelés dans de l'isopentane maintenu à -40°C par de la carboglace. Les divers cerveaux sont ensuite sectionnés à l'aide d'un cryostat en coupes de 20 $\mu$m d'épaisseur. Ces coupes sont étalées sur des lamelles de verre qui sont ensuite rapidement séchées à l'air froid. Les lamelles, assemblées sur un cadre, sont ensuite incubées pendant 10 minutes dans un tampon PIPES 50 mM, pH 7.4, maintenu à 0°C par de la glace. Les lamelles sont ensuite trempées pendant 20 minutes dans le même tampon, contenant en plus 200 nM de XA radioactif. Après trois bref lavages de chacun 10 secondes dans le tampon PIPES dépourvu de ligand radioactif, les coupes sont séchées par un courant d'air froid. Elles sont ensuite exposées à l'obscurité contre un film sensible au tritium. Après 2 mois d'exposition dans des cassettes hermétiques, les films dont développés et les niveaux de gris sont numérisés et comparés à une échelle de radioactivité tritium arbitraire, calibrée en Curie par gramme d'équivalent tissu (Amersham). Les résultats, qui représentent à un facteur près, la répartition des densités des récepteurs XA dans les diverses régions du cerveau de rat, sont données dans le tableau ci-joint.

Distribution régionale des sites de liaison du [3H] Xanthurénate. (Coupes sériées de 20 $\mu$m de cerveau de Rat Wistar).

| Structure cérébrale | Valeur densitométrique intégrée nCi / g t SD (n=3) | | |
|---|---|---|---|
| Noyau caudé partie latérale | 195 t 3.1 | 1942.6 t 30.3 | 252.0 t 4.0 fmol /mg |
| Substance noire compacte (A9) | 126 t 2.0 | 806.5 t 13.8 | 104.6 t 1.8 |
| Noyau interpédonculaire | 127 t 4.3 | 822.9 t 17.3 | 106.8 t 2.2 |
| Noyau amygdale centrale | 194 t 4.3 | 1921.3 t 42.9 | 249.4 t 5.6 |
| Hippocampe dorsal | 203 t 9.8 | 2068 t 41.1 | 268.5 t 5.3 |
| Hippocampe ventral | 128 t 2.1 | 847.5 t 16.9 | 110.0 t 2.2 |
| Noyau thalamus médiodorsal | 172 t 3.5 | 1568.8 t 39.3 | 203.7 t 5.1 |
| Noyau thalamus median post. | 180 t 7.5 | 1701 t 69.9 | 220.9 t 9.0 |
| Hypothalamus dorsomédian | 180 t 6.3 | 1700 t 73.2 | 220.7 t 9.5 |
| Aire tegmentale ventrale (A10) | 119 t 7.5 | 691.8 t 43.6 | 89.8 t 5.6 |
| Noyau accumnbens latéral | 193 t 10 | 1904.9 t 99.0 | 247.3 t 12.8 |
| Noyau raphé dorsal (B7) | 121 t 10 | 724.5 t 59.8 | 94.1 t 7.7 |
| Noyau raphé médian (B8) | 110 t 2.0 | 544.2 t 16.9 | 70.6 t 2.2 |
| Lobules cérébelleux | 180 t 3.5 | 1740.9 t 58.5 | 226.0 t 7.6 |
| Cortex pariétal | 184 t 1.4 | 1757.3 t 23.3 | 228.2 t 3.0 |
| Cortex temporal | 115 t 7.7 | 634.4 t 52.3 | 82.4 t 6.8 |
| Cortex cingulaire partie ant. | 180 t 5.2 | 1691.8 t 49.5 | 219.7 t 6.4 |
| Cortex préfrontal | 180 t 11.5 | 1691.8 t 108.2 | 219.7 t 14.0 |
| Gris périaqueducal | 125 t 5.65 | 724.5 t 40.0 | 94.1 t 5.2 |
| Cortex pyriforme | 186 t 7.7 | 1798.3 t 91.7 | 233.5 t 11.9 |
| Bulbes olfactifs | 181 t 7.0 | 1703 t 81.5 | 221.2 t 10.6 |
| Medulla oblongata | 149 t 4.9 | 1249.1 t 50.7 | 162.2 t 6.6 |

| Globus pallidus | 163 t 7.7 | 1413.2 t 67.2 | 183.5 t 8.7 |
|---|---|---|---|
| Noyau septum latéral | 175 t 6.1 | 1609 t 55.7 | 208.9 t 7.2 |
| Noyau septum médian | 147 14.9 | 1150 t 116.3 | 149.4 t 15.1 |
| Cortex occipital | 152 t 51 | 1232 t 291.7 | 160.0 t 37.8 |

**5. ETUDE DE LA MODULATION PAR LE XA DE L'ACTIVITE DOPAMINERGIQUE NIGRO-STRIEE ET MESO-CORTICO-LIMBIQUE.**

**Matériels et Méthodes**

- **Animaux :** Rats males adultes Wistar pesant 250 à 275 g, hébergés par deux dans des cages plastiques avec une illumination standard de 7h à 19 heures et ayant un accès libre à l'eau et à la nourriture. Les études sur ces animaux ont été menées en accord avec les règlements du Conseil des Communautés Européennes du 24 novembre 1986 (86/609/EEC).

- **Produits chimiques:** XA; 6-OHDA hydrobromide; 2,4,5-trihydroxyphenylethylamine et desipramine ont été obtenus chez Sigma). Le NCS-486 à été fabriqué par le Laboratoire de Pharmacochimie du CNRS, Strasbourg.

- **Protocole chirurgical:** Une canule en 1, avec une extrémité de 4 mm,

- (CMA 12, Camegie, Suéde) a été utilisée pour les expériences, . La membrane de dialyse, de diamètre 500 $\mu$m, est constituée de polycarbonate-polyether avec un seuil d'arrêt à 20 Kda. Le guide de sonde est implanté dans le CPF sous repaire stéréotaxique, les animaux étant anesthésiés par du chlorhydrate de kétamine (150 mg / kg / i.p.)

- **Lésions à la 6-hydroxydopamine des noyaux dopaminergiques A9 et A10**
  Les animaux sont anesthésiés à la Kétamine (100 mg /kg i.p.). La 6-OHDA est dissoute dans du sérum physiologique contenant 0.01% d'acide ascorbique à la concentration de 4 $\mu$g / $\mu$l. Un $\mu$l sont injectés en deux minutes, sous repaires stéreotaxiques, à l'aide d'une seringue de Hamilton dans A9/A10. Après injections, on attend la diffusion de la toxine pendant une minute. Les neurones noradrénergiques sont protégés par l'injection préalable à l'animal de desipramine 25 mg/ kg i.p., 45 minutes avant la 6-OHDA.
  4 semaines après, les rats sont administrés par 1 mg/kg d'apomorphine en injection sous-cutanée et le nombre de tours controlatéraux effectués 15 minutes après l'injection, sont enregistrés.
  Les rats lésés ont effectué 130 $\pm$ 45 tours / 15 minutes. Six semaines après l'injection locale de 6-OHDA dans les nayaux dopaminergiques A9/A10 droits, les rats présentant une bonne altération fonctionnelle ont été sélectionnés et implantés pour des études en microdialyse.

- **Protocole de microdialyse**
  Les expériences ont été réalisées sur des rats conscients lésés et non lésés, 24 à 48 heures après l'intervention chirurgicale (mise en place du guide de microdialyse). La composition du milieu de microdialyse est : NaCl 147 mM; CaCl2 1,2 mM; MgCl2 1,2 mM; KCl 4,0 mM, pH 6,5. La vitesse de dialyse est de 1 $\mu$l / min (pompe CMA 100, Camegie). Les dialysats sont collectés toutes les 20 minutes et immédiatement stockés dans l'azote liquide jusqu'à l'analyse.

- **Analyse de la dopamine, DOPAC et HVA dans les liquides de microdialyse**
  Ces composés sont analysés par HPLC avec détection électrochimique. Le système chromatographique consiste en une colonne 25 cm X 4.6 mm C18 maintenu à une température constante de 30°C. La phase mobile est constituée de NaH2PO4 50 mM contenant 0.1 mM EDTA et du méthanol 6%, le pH de la solution étant réglé à 4.85.

- **Rendements in vitro**
  Le rendement in vitro pour le dialyse de la dopamine a été préalablement mesuré à 16% à la température du laboratoire et pour une vitesse de dialyse de 1$\mu$l / min.

- **Histologie**

  Après les expériences, le placement correct de la sonde est toujours vérifié par examen histologique post-mortem du cerveau de l'animal, après fixation dans du paraformaldéhyde.

- **Analyses statistiques**

  Les analyses de microdialyse ont été évaluées statistiquement par un ANOVA suivi d'un test de comparaison multiples de Newman-Keuls.

**RESULTATS**

**• Effet / dose du XA infusé localement dans le CPF sur la modification des concentrations extracellulaires de dopamine.**

Les concentrations de XA retrodialysées ont été respectivement de 1 µM (cercles noirs),de 5 µM (carrés noirs) et de 20 µM (triangles noirs). L'application de XA se fait pendant une dialyse de 20 minutes. Les résultats sont exprimés en pourcentage de la libération moyenne de base pour la dopamine (8 fractions de 20 min avant la stimulation par le XA), Figure 20.

La plus faible concentration en XA (1µM) ne modifie pas la libération de dopamine. Par contre, la concentration de 5 µM (environ 1.5 µM de XA dans le tissu cérébral selon les données du rendement in vitro) fait augmenter la libération extracellulaire de dopamine d'environ 250%. La dose de 20 µM (environ 6 µM dans le tissu) fait augmenter la dopamine à 400-450%.

**• Effets de l'application locale de 20 µM de XA dans le CPF sur les taux de dopamine, DOPAC et HVA.**

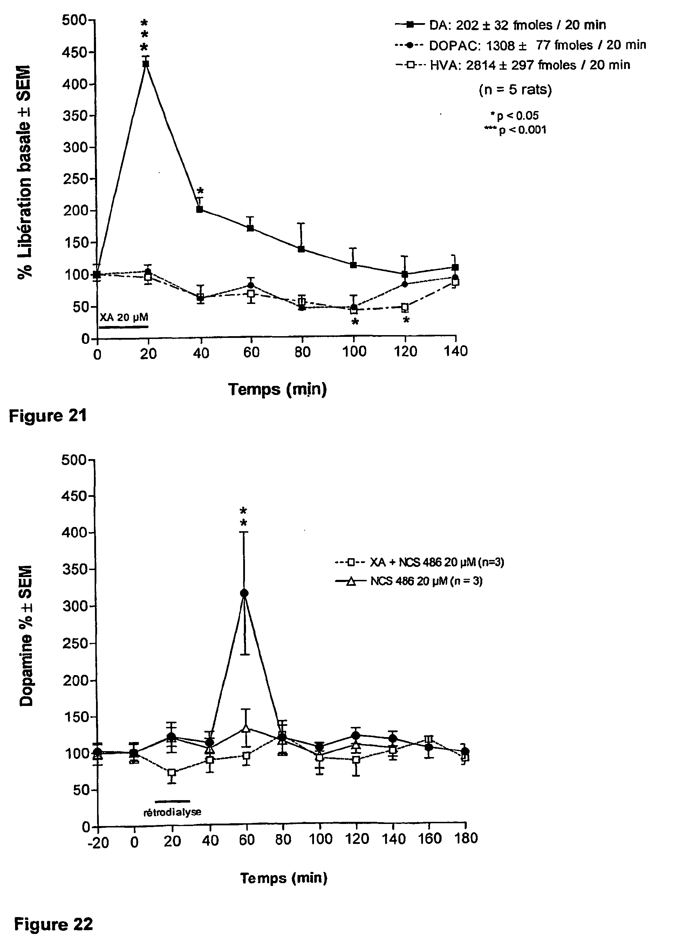

Les résultats sont exprimés en pourcentage de dopamine libéré par rapport à la ligne de base (8 x 20 min de dialyse avant l'injection du XA), Figure 21. Les carrès noirs représentent la dopamine, les cercles noirs représentent le DOPAC et les carrés blancs représentent l'HVA. Dans ce cas, l'augmentation de dopamine est, comme précédement, de 400-450% du niveau basal après 20 minutes, mais les taux de DOPAC et d'HVA baissent légèrement au bout de 100 à 120 minutes.

**• Effets de l'application locale de 20 µM de XA dans le CPF sur la libération extracellulaire de dopamine, en présence ou en absence de 20 µM de NCS-486.**

Les résultats sont exprimés en pourcentage de la libération basale de dopamine (moyenne de 8 x 20 minutes fractions de dialysats avant le traitement avec XA (Figure 22). Application de 20 µM de XA pendant 20 minutes (cercles noirs), ou co-infusion de XA 20 µM + NCS 486 20 µM (carrés blancs) ou application de 20 µM de NCS-486 seul (triangles blancs). On peut observer que le traitement avec NCS-486 seul ou NCS-486 + XA ne modifient pas la libération de dopamine, par contre XA seul augmente cette libération de 300% environ, 40 minutes environ après la stimulation.

**6. ETUDES ELECTROPHYSIOLOGIQUES**

**Enregistrements électrophysiologiques**

Les cellules de la lignée neuronale NCB-20 étaient enregistrées grâce à la technique du patch-clamp (Hamill et al., 1981) dans sa configuration dite cellule attachée. Les milieux d'enregistrements étaient conçus tel que seule l'activité des canaux cationiques aspécifiques et des canaux à chlorure se trouvant dans le fragment de membrane sous la pipette, pouvait être enregistrée de manière isolée. Les courants spécifiques calciques, sodiques et potassiques étaient minimisés par substitution de la majorité des cations du milieu pipette, par un cation imperméant la N-methyl-D- glucamine (NMDG) ou blocage par du TEA (Tétra-Ethyl-Ammonium). La substitution des cations perméants dans la pipette par la NMDG permettait aussi d'avoir les courants cationiques aspécifiques essentiellement dans le sens sortant la composante entrante étant minimisée. En outre, dans certaines expériences l'activité des canaux à chlorure était enregistrée uniquement dans le sens entrant grâce au remplacement des ions chlorure du milieu pipette par du TCA (acide trichloracétique). Le contrôle du potentiel de membrane cellulaire à une valeur proche de zéro mV se faisait en utilisant un milieu extracellulaire KCl. Ainsi le potentiel du fragment de membrane enregistré correspond à l'opposé du potentiel imposé dans la pipette d'enregistrement. Dans ces conditions les solutions d'enregistrement avaient la composition suivante : (en mM) : NMDG/CI(ou TCA) 140, KCl 2, $MgCl_2$ 1, HEPES 10, TEA/Cl 15, pour le milieu pipette et NaCl , KCl 141, $CaCl_2$ 0.5, HEPES 10, EGTA 5, pour le bain, le pH était ajusté dans les deux cas à 7.4 avec respectivement du TRIS base et KOH.

Les produits chimiques provenaient de chez Sigma (Saint Quentin Fallavier, France).

Après réalisation d'un 'gigaseal' avec des pipettes d'une résistance comprise entre 4 et 7 MΩ, les courants ioniques étaient mesurés grâce à un amplificateur de patch-clamp (EPC-7 amplifier, List-Medical, Darmstadt, RFA ou Axopatch B200, Axon Instruments, CA). L'acquisition du signal se faisait ensuite (Fréquence d'acquisition de 1 à 5 kHz) à l'aide d'une carte interface (Scientific Instruments, OH) et du logiciel pClamp 6 (Axons Instruments, CA). Les produits étaient dilués à la concentration voulue dans le milieu extracellulaire. L'application se faisait par gravité à travers un système de perfusion multivoies chacune d'elles ayant un diamètre interne de 300 μm. Le changement de solution s'opérait en plaçant après ouverture du robinet la voie correspondante en face de la cellule enregistrée.

## Résultats

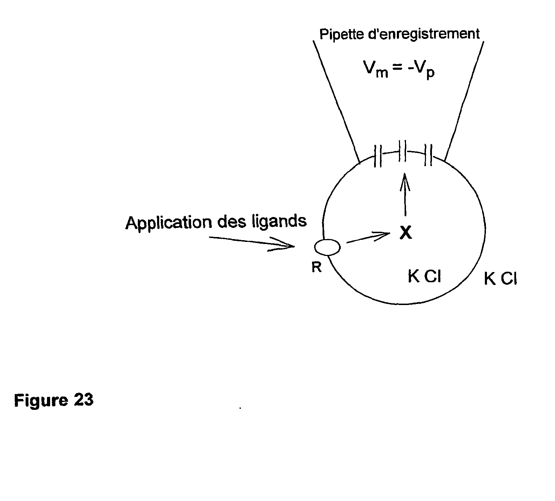

Les cellules NCB-20 expriment un site de liaison qui reconnaît spécifiquement l'acide xanthurénique. Le but de ces expériences fonctionnelles était de détecter tout phénomène membranaire électrogénique pouvant être activé par le(s) récepteur(s) de l'acide xanthurénique. Certains arguments laissaient à penser que ce récepteur est couplé aux protéines G et donc qu'un effet membranaire passerait par un relais intracellulaire. Nous avons en conséquence prospecté une éventuelle action sur les perméabilités cationiques et chlorures dont l'activation dépend à la fois du potentiel de membrane et de facteurs cytosoliques (essentiellement l'ion calcium et les protéines kinases; Evans and Marty, 1987; Taleb et a/.,1988; Leech et *al.*, 1996). Ces deux types de canaux ioniques ont donc été enregistrés de manière isolée (voir méthodes) et selon la mise en jeu de l'une ou l'autre des deux perméabilités. Le potentiel d'inversion de la réponse serait compris entre -49.5 ou - 11.8 mV respectivement pour une réponse cationique (cations monovalents) ou chlorure. Dans ces conditions tout changement au niveau des facteurs cytoplasmiques sera révélé par les paramètres de la relation courant potentiel (Figure 23).

Les cellules étaient enregistrées en cellule-attachée. Le courant traversant les canaux insérés dans la membrane sous la pipette était enregistré de manière isolée. Le potentiel de la membrane cellulaire était imposé à 0 mV par une concentration de KCl dans le bain équivalente à celle du milieu cytoplasmique. Dans ces conditions le potentiel du fragment de membrane sous la pipette est opposé à celui de la pipette.

### Effet de l'acide xanthurénique sur l'activation du courant cationique

Le fragment de membrane enregistré était périodiquement stimulé (fréquence 0.2 Hz) par une rampe de potentiel de -70 à 100 mV (voir protocole dans la Fig.24 A, trace du bas). Dans les conditions contrôles utilisant un milieu pipette pauvre en ion chlorure (substitués par du TCA), le courant enregistré était stable et avait une amplitude de -0.4 ± 0.1 et 8.6 ± 0.2 pA (n = 30) aux potentiels respectifs de -70 et 100 mV. Ce courant s'inversait à un potentiel de -48.3 ± 3.5 mV (n = 3). Cette valeur proche du potentiel d'inversion des cations monovalents (-49.5 mV, si on estime que la concentration intracellulaire des ions $K^+$ et $Na^+$ est respectivement de 140 et 2 mM) montre dans ces conditions d'enregistrement, qu'il y a une perméabilité basale sélective aux cations. La relation courant potentiel présentait une forte rectification sortante (Fig.24A et C), en accord avec la distribution asymétrique des cations de part et d'autre du fragment de membrane enregistré. En présence de l'acide xanthurénique à une concentration de 1 à 20 μM après un délai (de 1 à 4 min) dépendant de la concentration de l'agoniste, l'amplitude du courant cationique est augmentée (de 10 à 40 %) avec une cinétique lente sur lequel peut se greffer des courants transitoires de grande amplitude. Dans le cas de la fig.24, l'augmentation de l'amplitude du courant au pique du transitoire et au potentiel de 100 mV était d'un facteur 5.

En moyenne, pour une concentration de 20 μM de l'agoniste, l'amplitude du courant est augmentée d'un facteur 5.0 ± 1.7 (n=3). La relation I-V du courant spécifiquement active en présence de l'acide xanthurénique (après soustraction du courant obtenu en conditions contrôle) a des caractéristiques similaires à celle du courant contrôle (Fig.24C). En particulier, le potentiel d'inversion était quasiment identique à celui du contrôle et avait d'une valeur moyenne de -51.6 ± 2.1 (n=3). Ceci est vrai à la fois pour le courant lent et pour le courant transitoire. Cette valeur du potentiel d'inversion est voisine du potentiel d'équilibre des cations monovalents ce qui suggère que l'acide xanthurénique peut induire dans ces conditions, l'activation d'un courant de nature cationique. Le fait que ce courant soit enregistré au niveau d'un fragment de membrane physiquement isolé du reste de la cellule où l'agoniste est appliqué, montre que l'activation de ces canaux cationiques passe par un relais intracellulaire intermédiaire entre le récepteur et le canal.

Pour répondre à la question si ces canaux cationiques laissent passer aussi les cations divalents, en particulier le calcium, nous avons réalisé le même type d'expérience que précédemment mais avec un milieu pipette contenant essentiellement des ion $Ba^{2+}$ (l'ensemble des cations monovalent est remplacé par du $Ba^{2+}$ à l'exception de 2 mM d'ions $K^+$ et 15 mM d'ions $TEA^+$). Dans ces conditions aussi on observe en présence de l'acide xanthurénique, l'activation d'un courant dont le potentiel d'inversion avait cette fois ci une valeur moyenne de 2.6 ± 2.5 mV (n=7). La substitution des cations monovalents par le $Ba^{2+}$ a déplacé le potentiel d'inversion de la réponse d'environ 54 mV. Dans le cas où les ions $Ba^{2+}$ ne participeraient pas au courant activé par l'agoniste, le potentiel d'inversion attendu aurait une valeur de -53.5 mV. La différence observée témoigne du passage des ions $Ba^{2+}$ à travers les canaux cationiques.

**Effet de l'acide xanthurénique sur l'activation d'un courant chlorure**

Sur un certain nombre de réponses en particulier celles qui ne présentent pas de courant transitoire, on a observé cependant une évolution du potentiel d'inversion du courant induit par l'agoniste. Ainsi dans le cas illustré dans la Fig.25, le début de la réponse avait un potentiel d'inversion de -16.5 mV qui témoigne de la contamination du courant cationique par un autre courant dont le potentiel d'équilibre a une valeur plus dépolarisée. Dans une deuxième phase de la réponse le potentiel a évolué vers une valeur encore plus dépolarisée -4.2 mV. le courant qui s'est rajouté a un potentiel d'inversion compatible avec un mouvement d'ions chlorure dans nos conditions expérimentales.

**Pharmacologie de la réponse à l'acide xanthurénique**

Le NCS-482 appliqué à une concentration de 10 à 20 $\mu$M à la cellule NCB-20, induit une réponse quasi-identique à celle de l'acide xanthurénique (Fig.25 et 26). Ce résultat, montre que le NCS-482, une molécule dérivé de l'acide xanthurénique capable de déplacer la liaison de l'acide xanthurénique, a des propriétés d'agoniste sur le récepteur du Xanthurénate. Le NCS-486 qui est un autre dérivé de l'acide xanthurénique et qui déplace également la liaison de l'acide xanthurénique, n'a aucune action quand il est appliqué seul (20$\mu$M) par contre sa présence réduit l'amplitude de la réponse induite par l'agoniste (Fig.26). Ce qui lui confère des propriétés antagonistes sur le récepteur du xanthurénate.

**7. NEUROPHARMACOLOGIE DE L'ACIDE XANTHURENIQUE (XA) EVALUATION COMPORTEMENTALE CHEZ LE RAT.**

**1) Mesure de l'activité locomotrice par cellules infra-rouges :**

Les doses étudiées ont été les suivantes : 12.5, 25, 37.5, 50, 100 et 200 mg/kg (i.p.) (Figure 27). Ce test a mis en évidence une diminution de l'activité motrice, et ceci à des doses de XA supérieures ou égales à 50 mg/kg. L'intensité de la sédation observée a été proportionnelle à la quantité de produit injectée.

**2) Mesure de la réactivité globale de l'animal par le test « open field ».**

Ce test a confirmé l'absence de sédation à la dose de 37.5 mg/kg, dose à laquelle l'exploration du centre de la cage était significativement augmentée, ceci pouvant être interprété comme un effet anxiolytique potentiel (Figure 28). Cette tendance à été confirmée par un test évaluant les interactions sociales entre deux congénères (*Ramos et al. Behav Brain Res 85 1997 57 - 69).* La dose de 50 mg/kg a montré un effet significatif de l'augmentation de la durée des interactions sociales de l'animal traité par rapport à son congénère témoin non traité. A la dose de 100 mg/kg, la sédation significative induite par le produit a perturbé cette évaluation.

**3) Mise en evidence d'un effet de type antidépresseur par le test de Porsolt.**

Les doses utilisées ont été les suivantes : 50, 100, 150 et 200 mg/kg i.p. Ce test a été validé par l'Imipramine 30 mg/kg. Le test utilisé est le test de Porsolt et al. Eur. J Pharmacol 47 (1978) 379 - 391.
Les doses de 100, 150, 200 mg/kg ont montré une diminution statistiquement significative des durées cumulées d'immobilité indiquant un effet similaire à celui obtenu par l'Imipramine (Figures 29 et 30).

**4) Mise en évidence d'un effet de l'ac xanthurénique sur les processus de mémorisation par le test de reconnaissance d'objets.**

Ennaceur et Delacour ; Behav Brain Res 31 (1988) 47 - 59.
Les doses utilisées ont été les suivantes : 37.5 et 75 rnglkg i.p.
Les résultats obtenus (Figure 31) ont montré une augmentation significative de l'indice de reconnaissance des objets par les animaux traités par rapport aux contrôles. L'augmentation de cet indice peut être interprétée comme un apport bénéfique du produit favorisant la mémoire à court terme.

**5) Effets de l'ac. xanthurénique après injections intracérébrales.**

Le produit a été injecté par voie intra-cérébroventriculaire (i.c.v) d'une part, et dans des structures cérébrales définies d'autre part: aire tegmentale ventrale (A10), substance noire (A9) ou noyau accumbens. Pour les doses de 2, 4, 10, 50 $\mu$g/rat de XA, des stéréotypies proportionnelles à la dose injectée ont été observées. Après disparition des stéréotypies (30 minutes $\pm$ 15), la ré-injection du XA a conduit aux mêmes observations.

**6) Etude des potentialités antagonistes du NCS-486 sur des animaux dont les voies nigrostriatale et mésocor-ticolimbique ont été lésées de manière unilatérale par des injections intracérébrales (VTA ; SNc) d'un neurotoxique (6-OHDA) après protection des neurones noradrénergiques par prétraitement à la désipramine.**

L'injection d'une dose non sédative de XA (25 mg / kg i.p.) n'antagonise pas les rotations contralatérales induites par l'injection d'apomorphine (0.05, 0.1 mg /kg s.c.)
La recherche d'une potentialisation des rotations ipsilatérales induites par l'amphétamine et l'antagonisme éventuel du NCS-486 sur les rotations ipsilatérales induites par 25 mg / kg i.p. de xanthurénate est en cours.

**7) Analyse électroencéphalographique (EEG) de la sédation induite par l'injection (i.p.) d'ac. xanthurénique.**

Les doses étudiées se sont réparties entre 50 et 500 mg/kg (i.p). L'analyse E.E.G détaillée n'avait pas mis en évidence de tracé de type « épileptique » ou de type « sommeil lent profond ». Seule une réduction de l'amplitude des ondes EEG a été observée. Cette sédation était accompagnée d'une baisse significative de la température corporelle proportionnelle à la dose de produit injectée (- 0.5° à -2°C).
__EN CONCLUSION,__ les tests neuropharmacologiques que nous avons menés chez le rat après traitement à l'acide xanthurénique soit par voie périphérique, soit par voie locale, ont montré :
Le XA possède un effet sédatif chez l'animal, avec un effet / dose très démonstratif. Cet effet sédatif est corroboré par plusieurs tests. Dans l'open field où on observe le comportement spontané de l'animal, nous avons pu remarquer que le XA possédait un effet anxiolytique à des doses non sédatives et qu'il favorisait les interactions sociales. Certains tests visant à explorer un effet antidépresseur du XA ont montré que celui-ci potentialisait l'humeur de l'animal, suivant un effet / dose. Des tests mnésiques démontrent que le XA favorise la mémoire à court terme. Enfin, cette substance possède un fort pouvoir dopaminergique objectivé par l'induction de stéréotypies. Ces stéréotypies sont bloquées par l'antagoniste NCS-486 à fortes doses. L'examen EEG ne montre pas d'effets épileptiques du XA ou d'effet inducteur sur le sommeil lent profond, par contre on observe une diminution de l'amplitude du tracé et une hypothermie, ces deux phénomènes pouvant être corrélés à la sédation.

**8. Mise en évidence et caractérisation d'un système de transport pour le XA en culture de neurones.**

**1) Matériel et Méthodes**

*MISE EN CULTURE DES CELLULES NCB-20.*

Entretien de la souche cellulaire.
Les cellules NCB-20 sont conservées congelées dans l'azote liquide sous forme d'aliquotes provenant de la souche mère. Dans le cadre d'une étude, un aliquote est décongelé et mis dans les conditions de culture adéquate à l'étude. Pour entretenir la lignée, dès que la boite de culture cellulaire arrive à confluence, la boite initiale sert à ensemencer une autre boite en ne prélevant qu'une partie aliquote des cellules mis en suspension cellulaire dans la boite d'origine. Ainsi, les repiquages sont réalisés dans du milieu de DMEM contenant 10% de sérum de veau foetat (SVF) et la dilution est généralement une dilution au dixième.
Culture des cellules NCB-20 différenciées.
La culture des cellules est préparée grâce à une boite de culture de cellules NCB-20 confluente à 80%. Le milieu de culture est éliminé puis remplacé par 10 ml de DMEM contenant 10% de SVF. Les cellules sont alors décollées et mises en suspension par passages répétés du milieu à travers une aiguille de 2 mm de diamètre. Les cellules sont ensuite comptées dans un hémocytornètre. La suspension finale est ajustée à une densité cellulaire de $3.10^4$ cellules/mi de milieu DMEM contenant 10% de SVF et 1 mM d'AMPc. Les boîtes de pétri sont ensemencées avec 2 ml de cette suspension puis incubées à 37°C en atmosphère saturée d'humidité et en présence de 5% de $CO_2$. Les cellules sont utilisées 4 jours après leur mise en culture.

*MESURE DU TRANSPORT DE XA DANS LES CELLULES NCB-20*

A partir de cellules NCB-20 de 4 jours différenciées à l'AMPc, le milieu de culture est remplacé par une des solutions tampon décrites précédemment (Krebs avec ou sans $Na^+$, permettant d'appréhender respectivement le transport actif $Na^+$-dépendant et le transport passif qui relève principalement de la diffusion) selon l'expérience envisagée. Les boites de culture sont placées dans un bain-marie à 37°C pendant 10 minutes. Le tampon est ensuite remplacé par la même solution tampon contenant du $[^3H]XA$ et du XA à des concentrations variants de 1 à 500 $\mu$M. Le $[^3H]XA$ est présent à très faible concentration et ne joue qu'un rôle de traceur du XA froid (non radioactif). Après une minute d'incubation, les solutions sont aspirées et les cellules sont lavées 3 fois 10 secondes avec 1 ml du même tampon (Krebs avec ou sans Na⁺), maintenu dans la glace. Le transport actif nécessite de l'énergie (ATP) et est fortement ralenti à des températures inférieures à 37°C. Le tapis cellulaire lavé est congelé dans les boites puis, le lendemain, de l'eau distillée est ajoutée pour obtenir une solution de débris cellulaire contenant le radioactif intracellulaire ([³H]XA). La congélation permet de faciliter l'homogénéisation par des passages répétés de la solution à travers une pipette. Un aliquote (900µL) est ajouté à un liquide de scintillation (Rotiszint®, 4 ml). Le reste de la solution est prélevé pour effectuer un dosage des protéines selon la méthode de BCA (Uptima). La quantité de radioactivité mesurée par le compteur à scintillation est convertie grâce à un étalonnage approprié, en picomoles de XA capturée par les cellules/mg de protéines. On effectue la même manipulation pour chacune des boites contenant les cellules différenciées. Toutefois, à chaque expérimentation, un des paramètres est modifié afin de déterminer les caractéristiques cinétiques et pharmacologiques du transport de XA dans les cellules NCB-20.

Les conditions de transport sont mesurées en fonction :

➣ Du temps d'incubation:

Ces expériences permettent de définir la cinétique du transport du XA.

Pour chaque durée d'incubation choisie, on réalise la mesure du transport sur 9 boites de cellules NCB-20 avec Na⁺ et sans Na⁺. Dix temps différents sont choisis: 0-10-30 secondes et 1, 2, 3, 5, 8, 10, 12 minutes. Quatre expériences différentes ont été réalisées.

➣ De la concentration en [³H]XA :

Cette expérimentation permet de définir les paramètres cinétiques que sont la vitesse maximum du transport ($V_m$) et la concentration de XA nécessaire pour obtenir la moitié de cette vitesse maximum ($K_m$).

Pour chaque concentration de XA, on réalise la mesure de son transport sur 9 boîtes de cellules NCB-20 avec Na⁺ et 9 sans Na⁺. Sept concentrations différentes sont choisies: 1-5-12,5-25-50-100-200 µM. Quatre expériences distinctes ont été réalisées.

*DOSAGE DES PROTEINES*

Le dosage des protéines est réalisé en microplaque. La microplaque doit contenir le nombre de puits nécessaire pour effectuer le dosage en double pour chaque boîte de culture et pour la gamme d'étalonnage.

Les cellules contenues dans le fond de chaque boîte de culture traitée est repris par 1 ml d'eau bi-distillée. Après plusieurs aspiration-refoulements pour décoller tous les débris cellulaires et homogénéiser la suspension. Deux prises d'essai de 20 µl en sont ensuite prélevées et déposées dans les puits de la microplaque.

Un espace est réservé pour la gamme de calibration comprise entre 0,032 et 2 mg/ml. Chaque suspension de débris cellulaire est mesurée en double. 200 µl de réactif (mélange de 50 parties du réactif A et d'une partie du réactif B) sont ensuite ajoutés dans chaque puits. Après agitation la plaque est mise à incuber 30 min à 37 °C.

*COMPTAGE DE LA RADIOACTIVITE*

4 ml de scintillateur sont ajoutés dans les tubes contenant 900 µl de la suspension cellulaire obtenue. Le tube témoin est réalisé avec 900 µl d'eau bi-distillée, et les tubes permettant la mesure de l'activité spécifique contiennent 10 µl des solutions radioactives utilisées comme réactif lors de la manipulation et 890 µl d'eau bidistillée. Les tubes sont fermés, vortexés puis laissés une nuit à 4°C avant d'être mesurées dans le compteur à scintillation. Ce comptage permet de mesurer la quantité de radioactivité intracellulaire qui est directement proportionnelle à la quantité de XA incorporé dans la cellule par transport passif ou actif. Pour obtenir la quantité de transport actif, il suffit de soustraire au transport total (Krebs avec Na⁺) le transport passif (Krebs sans Na⁺).

**2) Résultats concernant le transport de XA**

La totalité des calculs a été réalisée par ordinateur à l'aide du programme Excel. Les résultats sont alors traduits sous forme graphique à l'aide d'un autre programme Prism 3.0 qui nous permet également le calcul des équations de droite et des statistiques.

Les résultats sur le transport de XA dans les cellules NCB-20 en fonction du temps sont donnés figure 32.

Lors de l'étude du transport en fonction du temps d'incubation des cellules en présence de XA, on peut observer que les trois courbes ont une allure similaire. Le transport est augmenté d'environ 20% en présence de sodium. Les 3 courbes saturent à partir d'1 minute environ, ce qui nous permet de définir le temps optimum de mesure du transport du XA dans les cellules NCB-20. Pour la suite des expériences, ce temps de 1 minute sera utilisé.

Les résultats du transport de XA dans les cellules NCB-20 en fonction de la concentration en [³H]XA sont donnés à la figure 33.

La représentation de Scatchard permet de déterminer les paramètres mathématiques du transport intracellulaire de XA soit un Km de 105 $\mu$M et une Vmax de 1229 pmoles/mg protéine/ minute.

Lors de l'étude du transport de XA en fonction de la concentration de XA, on met en évidence une saturation du transport et donc du transporteur qui mathématiquement se traduit par la valeur de la vitesse maximale de transport. Dans nos expériences, cette donnée est de Vmax =1229 $\pm$ 440 pmoles/mg protéines . min. Une autre donnée mathématique caractéristique du transport est le Km qui représente la concentration de XA nécessaire pour une vitesse équivalente à la moitié de la vitesse maximale. Dans nos expérimentations, le Km est de 105 $\pm$ 81 $\mu$M.

La concentration optimale en XA pour l'étude pharmacologique de cette molécule sera de 100 $\mu$M, ce qui correspond au Km qui a été déterminé.

Interférence du transport de XA avec d'autres molécules endogènes

D'autres molécules endogènes, susceptibles d'être transportées par le transporteur XA, ont été testées. De plus, pour mettre en évidence le caractère énergie-dépendant de ce transport, nous avons bloqué la production d'énergie cellulaire par le deoxyglucose qui inhibe la glycolyse. Les résultats figurent sur l'histogramme de la figure 34, en pourcentage de capture par référence au transport en présence de XA radioactif seul (=100 %).

Il s'avère que le L-tryptophane, la L-tyrosine et l'acide kynurénique sont de bons inhibiteurs du transport cellulaire du XA. Il peut donc être envisagé que la capture neuronale de XA emprunte le transporteur d'acides aminés neutres. La présence d'ions cuivre ou d'ions zinc dans le milieu potentialise de façon très significative le transport de XA (trois fois plus pour les ions cuivre). La présence du poison cellulaire 2-deoxyglucose diminue le transport d'environ 50%, ce qui indique que ce transport nécessite une énergie cellulaire intacte.

## 3) Conclusions aux expériences de transport de XA

Les différentes expériences réalisées ont permis de mettre en évidence la présence d'un transport actif de XA dans les cellules NCB-20. Ce transport est un phénomène actif qui nécessite la présence d'ions sodium dans le milieu réactionnel. Ce transport se caractérise par une cinétique assez rapide puisque la vitesse optimum se situe aux environ de 1 minute d'incubation. Les paramètres cinétiques de ce transport actif ont été mesurés et comportent une vitesse maximum de 1229 pmoles/mg protéines.min et un Km de 105 $\mu$M. Un certain nombre d'acides aminés, dont le tryptophane et la tyrosine, inhibe ce transport, ce qui suggère que le transporteur impliqué pourrait être un transporteur neuronal pour les acides aminés neutres et que le transport de XA interfère avec le transport d'acides aminés essentiels pour la synthèse des catécholamines et de la sérotonine. Les composés synthétiques, ligands du récepteur XA, pourront être testés sur ce transporteur pour vérifier leurs spécificités.

## 9. Organisation et rôle du système XA dans la régulation de l'activité dopaminergique.

### 1) Le XA est-il co-libéré avec la dopamine au niveau des mêmes terminaisons lors de la stimulation des noyaux dopaminergiques A$_9$-A$_{10}$ ?

Pour répondre à cette question, on a étudié les modifications de la libération de dopamine et de XA extracellulaire après lésion des noyaux dopaminergiques à la 6-hydroxydopamine (6-OHDA). Cette lésion détruit en particulier les terminaisons dopaminergiques du cortex frontal. On a, d'autre part exploré les modifications de libération de glutamate et de GABA sous l'influence de XA pour essayer de comprendre la séquence des évènements menant à la modification de libération de la dopamine au niveau frontal chez le rat.

Protocole utilisé :

La voie dopaminergique mésencéphalique a été lésée de façon unilatérale par injection stéréotaxique de 6-hydroxydopamine hydrobromide (6-OHDA Hbr, Sigma) dans l'aire tegmentale ventrale (VTA) et dans la substance noire compacte (SNc). L'intervention a été pratiquée dans un cadre stéréotaxique (Narishige) sur des rats de souche Wistar sous anesthésie (Imalgène 100 mg / kg i.p.). La neurotoxine (6-OHDA) a été dissoute dans une solution de chlorure de sodium isotonique (NaCl 0.9%) contenant 0.01% d'acide ascorbique à une concentration finale de 4$\mu$g de base libre / $\mu$l et 6 $\mu$g (1.5 $\mu$l / point d'injection) ont été injectés en deux minutes en utilisant une canule d'injection en acier inox de diamètre 20 G. En accord avec l'atlas de Paxinos et Watson, les coordonnées stéréotaxiques utilisées sont exprimées en millimètres par rapport au bregma : AP : 2.3 ; ML : 0.5 ; DV : 8.7 mm et AP : 2.3 ; ML : 2.0 ; DV : 7.5 mm, respectivement pour le VTA et la SNc. La coordonnée dorso-ventrale à été prise à partir de l'os crânien, les barres d'oreilles étant positionnées à 3,3 mm en dessous de la barre des incisives supérieures. Après l'injection totale du volume à la vitesse de 1 µl / minute (pompe CMA 100) l'aiguille d'injection est restée en place une minute supplémentaire, afin de laisser s'effectuer la libre diffusion de la toxine autour du point d'injection. Les neurones noradrénergiques ont été protégés de cette lésion par une injection préalable de désipramine à raison de 25 mg / kg i.p. au temps 45 minutes avant l'injection de 6-OHDA.

Sélection des animaux

Quatre semaines après la lésion, les rats ont été testés après administration sous-cutanée d'un agoniste direct (apomorphine 0,1 et 1,0 mg / kg) et le nombre de rotations controlatérales (côté opposé à la lésion) a été enregistré pendant 15 minutes. Pour les rats lésés, le résultat a été le suivant: 130 ± 45 tours / 15 minutes. (n = 12 animaux).

Six semaines après les injections dans l'aire tegmentaire ventrale et la substance noire compacte droites, les rats ayant donné satisfaction au comportement de rotations controlatérales induites par l'apomorphine ont été sélectionnés et implantés pour effectuer les expériences de microdialyse.

A la fin des expériences, les animaux utilisés ont été euthanasiés. Le striatum et la région des noyaux mésencéphaliques ipsilatérale et controlatérale ont été prélevés et analysés par HPLC pour leur teneur résiduelle en dopamine.

Les résultats exprimés en pmoles / g de tissu frais ± SEM (n=3) ont été les suivants :

Striatum lésé : 261 ± 123 versus striatum intact 9140 ± 1413 (- 98% p = 0.0033)

VTA - SNc lésé : 32 ± 31 versus VTA - SNc intact 563 ± 128 (- 95% p = 0.0157).

Ceci indique que la lésion des neurones dopaminergiques a été satisfaisante et qu'on observe une chute très importante de la teneur en dopamine du striatum et des noyaux dopaminergiques.

Mesure de la libération extracellulaire de la dopamine et de ses métabolites après lésion neurotoxique des noyaux dopaminergiques mésencéphaliques :

Les animaux lésés ont été implantés (sonde de dialyse et électrode bipolaire), stimulés électriquement au niveau du VTA (100 et 200 µA) et dialysés au niveau du cortex préfrontal / cingulaire antérieur suivant le même protocole que celui déjà décrit dans les expériences précédentes.

Parmi les paramètres mesurés (DA, DOPAC, HVA, XA et 5-HIAA), seuls le XA et le 5-HIAA ont été détectables sur les chromatogrammes.

Les résultats obtenus montrent que la stimulation électrique (100 et 200 µA) du VTA après lésion à la 6-OHDA des noyaux dopaminergiques (VTA et SNc) a pour conséquence de provoquer une libération frontale de XA (+ 151 ± 51% et + 667 ± 115% pour respectivemment une stimulation de 100 µampères et 200 µampères), accompagnée d'une libération de 5-HIAA sans modification du niveau de la libération de dopamine résiduelle après la lésion (DA, DOPAC et HVA).

Ces résultats démontrent que XA et dopamine ne sont pas co-libérés au niveau frontal, alors que le XA module la libération de dopamine. XA est donc libéré par des terminaisons probablement spécifiques et module la libération de dopamine via des récepteurs localisés au niveau des extrémités dopaminergiques, soit au niveau des noyaux dopaminergiques eux-mêmes (VTA et SNc), soit enfin au niveau de terminaisons contrôlant la libération de glutamate et/ou de GABA dans le cortex frontal. Les autoradiographies quantitatives ont montré l'existence de sites de fixation à haute affinité pour le XA, aussi bien dans le cortex frontal qu'au niveau des noyaux dopaminergiques du mésencéphale.

**2) Modification de la libération extracellulaire frontale en glutamate (GLU) et en GABA après rétrodialyse de XA 20 µM pendant 20 minutes**

L'objectif est ici de mettre en évidence une éventuelle modification de la libération de glutamate et / ou de GABA par le XA. Ces modifications auraient ensuite une répercussion sur la libération de dopamine. Dans cette hypothèse, les sites récepteurs XA se trouveraient situés sur des neurones glutamate et / ou GABA du cortex frontal.

a) Mesure des concentrations en acides aminés libres dans les dialysats

Les acides aminés contenus dans 15 µl de dialysat ont été séparés en fonction de leur degré d'hydrophobicité par chromatographie liquide haute performance (HPLC) en phase inverse. Ce système comprend un dégazeur (Waters In-Line Degasser), une pompe (Waters 626 Pump, Waters 600 S Controller), un collecteur-injecteur CMA/200 réfrigéré (Refrigerated Microsampler, CMA/microdialysis, Camegie) muni d'une boucle de 51 µl, une colonne nucleosil C18 (5 µm, 25 x 0,4 cm), un four (Waters), et un fluorimètre (Waters 470 scanning fluorescence detector). La phase mobile était constituée d'un gradient binaire entre une solution A : $NaH_2PO_4$ (Roth) 0,05 M 80% (pH 4.8 ajusté avec du NaOH 10 N), méthanol 20% (Chromanorm, Prolabo); et une solution B : $NaH_2PO_4$ 0,05 M 20% (pH 4.8 ajusté avec du NaOH 10 N), méthanol 80% et THF 5%. La mesure du glutamate et du GABA a été effectuée par détection fluorimétrique à 345 nm de longueur d'onde d'excitation et 455 nm de longueur d'onde d'émission après dérivatisation. L'acquisition des chromatogrammes ainsi que l'analyse quantitative des données ont été réalisées à l'aide du logiciel Millénium (Waters). La dérivatisation des échantillons a été faite par mélange de 15 $\mu$l de dialysat avec un volume de 15 $\mu$l de la solution de dérivatisation suivante : 7,5 mg d'o-phtaldialdéhyde (Sigma) dans 4,5 ml de tetraborate de Na 0,1 M pH 9.3, 500 $\mu$l de méthanol et 10 $\mu$l d'acide 3-mercapto-propionique (Sigma). L'élution a été fixée à 0,8 ml/min et 35°C suivant une succession d'étapes : 90% A et 10% B à 0 min; 40% A et 60% B à 15 min (gradient linéaire); 40% A et 60% B à 19 min (gradient isocratique); 0% A et 100% B à 19,1 min; 0% A et 100% B à 24 min (gradient isocratique); 90% A et 10% B à 24,1 min (gradient isocratique) et 90% A et 10% B à 30 min La limite de détection pour le glutamate et le GABA dans nos échantillons a été de 0,75 fmoles (0,05 $\mu$M). L'acide $\beta$-amino-isobutyrique (Sigma) a été utilisé comme standard interne.

Résultats obtenus :

La rétrodialyse de 20 $\mu$M de XA pendant 20 minutes au niveau du cortex frontal a pour conséquence de provoquer une baisse immédiate de la libération de GLU de 37% $\pm$ 1 pendant une durée de 90 minutes avant un retour progressif au niveau de base. Les résultats présentés en figures 35 et 36 sont exprimés en % $\pm$ SEM (n=2). La valeur 100 % (1nmole / 20 $\mu$l) représente la moyenne de 4 échantillons consécutifs avant stimulation.

**10. Répercussion de l'effet du stress induit par des chocs électriques intermittents sur la libération de noradrénaline (NE) et de XA au niveau du cortex préfrontal médian (CPF).**

Ce travail repose sur l'hypothèse que la noradrénaline (NE) préfrontale provenant du locus coeruleus (A6) joue un rôle important dans les différences comportementales individuelles de réactivité au stress. Le noyau A6 présentant un marquage de XA tritié sur les autoradiographies, nous avons voulu voir si la libération frontale de XA pouvait être impliquée dans les mécanismes de stress induit par chocs électriques chez l'animal.
L'activité locomotrice spontanée dans un nouvel environnement étant un indice comportemental de réactivité vis-à-vis d'une situation stressante, nous avons sélectionné des « animaux répondeurs » qui avaient une réponse comportementale plus importante dans ce test.
Les principales composantes de la réponse au stress étant la libération extra-hypothalamique de CRH (corticotropine releasing hormone) provoquant une stimulation de la tyrosine hydroxylase (TH) entraînant une augmentation de la noradrénaline au niveau du locus coerulerus et par conséquent une augmentation de la libération de NE au niveau des terminales (cortex préfrontal, l'amygdale et gyrus denté).

Protocole utilisé:

1) Sélection des animaux dans l'épreuve du « champ ouvert » :
Nombre de carreaux franchis dans une session de 5 minutes : 55.08 $\pm$ 22.6 (6.53) Moyenne $\pm$ SD (SEM) n = 12 rats de 350 g. de souche Wistar.

2) Stress et quantification neurochimique :
Le stress a été induit par des chocs électriques intermittents au niveau des pattes à raison de 1 choc de 0,3 mA / min pendant 20 minutes.

La libération de noradrénaline au niveau du cortex préfrontal a été mesurée en utilisant la technique de microdialyse intracérébrale sur animal vigile couplée à un système de chromatographie liquide à haute performance à détection électrochimique.

Résultats :

Les résultats sont donnés à la figure 37.
Pendant la durée du stress induit par des chocs électriques intermittents de 300 $\mu$A d'une durée de 1 seconde / min, pendant 20 min, la noradrénaline préfrontale a augmenté de +151 % par rapport à sa libération basale et celle du XA de +250 %.

Cette étude montre que la noradrénaline et XA sont libérés simultanément dans le cortex frontal de l'animal stressé. Le XA libéré peut représenter une réaction d'adaptation au stress et l'administration d'agonistes du récepteur XA dans des situations de stress pourrait représenter une nouvelle solution thérapeutique, d'autant plus que la dopamine libérée est très faible et que le XA et les agonistes du récepteur XA augmente cette libération.

**11. Caractérisations neuropharmacologiques des activités antagonistes au niveau du récepteur XA : étude d'un antagoniste intitulé NCS-486.**

**1) Etude des stéréotypies dopaminergiques induites par l'injection intracérébrale de XA et réversion par un antagoniste NCS-486.**

Les « stéréotypies » sont des mouvements involontaires effectués par l'animal après administration d'agonistes dopaminergiques directs (apomorphine) ou indirects (amphétamine).

Ces comportements stéréotypés sont les suivants : léchages, mâchonnements, hyperlocomotion, exploration, redressements, toilettages, enfouissements, étirements et reniflements.

Effets de l'acide xanthurénique

Les injections intracérébrales de XA au niveau du striatum, du VTA, des ventricules latéraux ou du noyau accumbens, induisent des stéréotypies dont l'intensité est proportionnelle à la dose injectée (2, 10, 50, 100 et 150 $\mu$g / rat). Ces comportements apparaissent deux minutes après injection intracérébrale et durent entre 30 et 40 minutes. Une heure après la disparition de celles-ci, ces stéréotypies peuvent être reproduites par une nouvelle injection. Après lésion neurotoxique des noyaux mésencéphaliques ($A_9$-$A_{10}$) à la 6-OHDA, l'injecüon de 25 mg / kg (i.p.) de XA ne modifie pas le nombre de rotations controlatérales induites par 0.1 mg / kg (s.c.) d'apomorphine. De la même manière, 25 mg / kg de XA ne modifie pas le nombre de rotations ipsilatérales induites par l'administration d'amphétamine.

Effets du NCS-486 sur les stéréotypies induites par le XA, l'apomorphine ou l'amphétamine :

2 $\mu$g/rat (i.c.v.) de NCS-486 antagonisent totalement les stéréotypies induites par 2 $\mu$g de XA (i.c.v.), alors que l'injection de NCS-486 seule (2 $\mu$g i.c.v.) ne modifie nullement le comportement de l'animal.
Injecté (i .p) ou (i.c.v.) le NCS-486 n'antagonise pas les stéréotypies induites par 0.5 mg / kg (s.c) d'apomorphine. Par contre, le NCS-486, à la dose de 200 mg / kg i.p, injecté 30 minutes avant le XA 2 $\mu$g / rat i.c.v. supprime ou réduit fortement l'apparition des stéréotypies.

**2) Etude du NCS-486 sur l'activité générale de l'animal mesurée dans le test de « l'openfield » et réversion de la sédation induite par le XA.**

Cette étude a montré qu'une heure après administration du NCS-486 (100 mg / kg p.o) celui-ci provoquait une hyperactivité de l'animal. De plus, le NCS-486 était capable d'antagoniser la sédation induite par une injection de 100 mg / kg (i.p.) de XA.
Les résultats obtenus sont rassemblés dans le tableau suivant :

| Items | Témoins | Traités NCS-486 | Variations en % |
|---|---|---|---|
| Toilettages | 1 $\pm$ 1 | 3 $\pm$ 1 | - |
| Sauts | 1 $\pm$ 2 | 2 $\pm$ 2 | - |
| Redressements | 20 $\pm$14 | 32 $\pm$ 1 | - |
| Carreaux totaux | 55 $\pm$ 37 | 143 $\pm$ 2 ** | +260 |
| Carreaux externes | 51 $\pm$ 33 | 128 $\pm$ 21* | + 251 |
| Carreaux internes | 4 $\pm$ 4 | 14 $\pm$ 1* | + 350 |
| Défécations | 2 $\pm$ 1 | 0 | - |
| Les résultats sont exprimés sous forme de moyenne $\pm$ SD (n=4 rats / groupe) Test de Student : * $p < 0.05$ ; ** $p < 0.01$ | | | |

Recherche d'une réversion de la sédation induite par l'administration de XA 100 mg / kg (i.p)
Les résultats obtenus sont rassemblés dans le tableau suivant :

| Items | Témoins | XA 100 mg/kg (i.p.) | XA 100 mg/kg (i.p.) + ND-7002 100 mg/kg (per os) |
|---|---|---|---|
| Toilettages | 4 ± 1 | 4 ± 2 | 4 ± 1 |
| Redressements | 66 ± 4 | 37 ± 14 | 51 ± 11 |
| Carreaux totaux | 193 ± 22 | 110 ± 25 * | 182 ± 15 |
| Carreaux externes | 153 ± 18 | 91 ± 17* | 148 ± 15 |
| Carreaux internes | 39 ± 9 | 19 ± 6 | 34 ± 8 |
| Les résultats sont exprimés sous forme de moyenne ± SEM (n=4 rats / groupe). Test de Student : * p < 0.05 | | | |

**12. Caractérisations des activités neuropharmacologiques de ligands se fixant sur le site allostérique pour l'acide kynurénique, partie intégrante du site récepteur de XA : exemple d'un composé, intitulé ND-7000, dérivé de l'acide xanthurénique ou de l'acide kynurénique.**

Effets du ND-7000 sur les stéréotypies induites par le XA.
Le ND-7000 injecté par voie intra-cérébroventriculaire à la dose de 20 μg / rat, n'induit aucune stéréotypie chez l'animal. A la dose de 50 μg/animal, le ND-7000 fait apparaître une sédation importante. En revanche, une injection simultanée de 10 μg de ND-7000 et de 10 μg de XA fait apparaître des stéréotypies. Une dose de 25 μg de ND-7000 + 25 μg de XA potentialise très nettement les stéréotypies (toilettages) et diminue le temps de latence d'apparition de ces mouvements involontaires (2 min au lieu de 5 min).

Etude du ND-7000 sur l'activité générale de l'animal dans le test de « l'openfield »

Le test, d'une durée d'observation de 15 minutes, a été effectué 30 minutes après injection du produit à la dose de 100 ou 200 mg / kg (per os).
Aux deux doses étudiées nous observons une diminution de l'activité globale des animaux qui est proportionnelle à la dose de produit injecté.
Les résultats obtenus sont rassemblés dans le tableau suivant :

| Items | Témoins | 100 mg/kg (i.p.) | 200 mg/kg (i.p.) |
|---|---|---|---|
| Toilettages | 5 ± 4 | 3 ± 2 | 4 ± 2 |
| Redressements | 53 ± 12 | 30 ± 6 (-44%) | 20 ± 9 (-63%) |
| Carreaux totaux | 215 ± 24 | 146 ± 15 (-33%) | 106 ± 22 (-51%) |
| Carreaux externes | 188 ± 35 | 129 ± 11 (-32%) | 97 ± 23 (-49%) |
| Carreaux internes | 27 ± 13 | 17 ± 8 (-38%) | 9 ± 7 (-68%) |

Les résultats sont exprimés sous forme de moyenne ± SD (n=5 rats/groupe).
Administré à la dose de 100 à 200 mg / kg (per os), le ND-7000 n'induit pas de catalepsie et ne modifie pas les activités réflexes mesurées dans des tests sensorimoteurs.
L'actographie obtenue avec administration de ND-7000 à 100 mg / kg per os (PO) est représentée à la figure 38.
En condusion, les effets de ND-7000 sur les activités dopaminergiques cérébrales induite par XA et sur la réactivité globale de l'animal mesurée dans l'open-field, sont en faveur de la potentialisation par ce produit de la fixation de XA sur son site récepteur. Ces résultats confirment les études de binding qui montraient une forte augmentation de la fixation de XA sur son site récepteur en présence de ND-7000. Ces expériences confirment au niveau pharmacologique, l'existence d'une autre voie pour potentialiser les effets de Xa. Il s'agit des ligands synthétiques se fixant sur le site allostérique. On peut imaginer également des effecteurs allostériques négatifs, antagoniste du binding de ND-7000 et des dérivés de l'acide kynurénique, qui diminueraient l'activité de XA en diminuant sa fixation sur son site récepteur. Ceci représente une nouvelle voie de recherche pour la synthèse de ligands originaux.

**13. Un exemple de ligand agoniste du récepteur XA, dérivé de l'acide xanthurénique, intitulé le ND-1301**

Le ND-1301 a été choisi pour illustrer la série des ligands agonistes du récepteur XA. Cette substance déplace le XA radioactif de son site avec une bonne affinité et induit une forte réponse électrophysiologique après étude en patch-clamp sur des cellules NCB-20. Le ND-1301, administré per os à l'animal, module également les taux de dopamine tissulaire cérébraux, comme l'indiquent les résultats ci-dessous.

L'effet dose/réponse après administration (P.O.) de ND-1301 sur la dopamine tissulaire dans différentes régions cérébrales est donné à la figure 39.

**Revendications**

1. Méthode in-vitro de sélection, identification ou caractérisation de composés modulant l'activité du système utilisant l'acide xanthurénique comme médiateur, comprenant la mise en contact in vitro ou ex vivo d'un composé test avec une cellule exprimant un récepteur naturel de l'acide xanthurénique ou une préparation membranaire présentant un récepteur naturel de l'acide xanthurénique et issue d'une cellule exprimant ledit récepteur, et la mise en évidence d'une liaison du composé test audit récepteur.

2. Méthode selon la revendication 1, comprenant la mise en contact d'un composé test avec ladite cellule ou ladite préparation membranaire, en présence d'un ligand marqué dudit récepteur et la mise en évidence d'une liaison du composé test par détermination du déplacement de la liaison du ligand marqué.

3. Méthode selon la revendication 2, **caractérisée en ce que** le ligand marqué est l'acide xanthurénique radiomarqué.

4. Méthode selon l'une des revendications précédentes, **caractérisée en ce que** la liaison du composé test au récepteur est mise en évidence par un effet biologique ou pharmacologique.

5. Méthode selon la revendication 4, **caractérisée en ce que** l'effet biologique ou pharmacologique mesuré est l'expression d'une ou plusieurs protéines cellulaires, l'expression d'un récepteur, l'activation d'un gène, l'internalisation du récepteur, l'apparition d'un courant électrique, d'un flux ou d'un influx d'ions.

6. Méthode selon la revendication 4 ou 5, comprenant la mise en contact d'un composé test avec une cellule exprimant un récepteur à l'acide xanthurénique, en présence d'un ligand dudit récepteur, la mesure d'un effet biologique ou pharmacologique caractéristique d'une liaison au récepteur de l'acide xanthurénique et la comparaison de l'effet mesuré à celui obtenu en l'absence de composé test.

7. Méthode selon l'une quelconque des revendications précédentes, **caractérisée en ce que** la liaison du composé test est mise en évidence par migration sur gel, électrophorèse, FRET SPA ou par détermination du déplacement d'un ligand marqué par le composé test.

**Claims**

1. An *in vitro* method for selecting, identifying or characterizing compounds which modulate the activity of the system using xanthurenic acid as a mediator, comprising contacting *in vitro* or *ex vivo* a test compound with a cell expressing a natural receptor of xanthurenic acid or a membrane preparation presenting a natural receptor of xanthurenic acid and derived from a cell expressing said receptor, and demonstrating a binding of the test compound to said receptor.

2. A method according to claim 1, comprising contacting a test compound with said cell, or said membrane preparation, in the presence of a labelled ligand of said receptor, and demonstrating a binding of the test compound by measuring the displacement of the binding of the labelled ligand.

3. A method according to claim 2, wherein the labelled ligand is radiolabelled xanthurenic acid.

4. A method according to anyone of the preceding claims, wherein the binding of the test compound to the receptor is determined by a biological or pharmacological effect.

5. A method according to claim 4, wherein the measured biological or pharmacological effect is the expression of one or more cellular proteins, the expression of a receptor, the activation of a gene, the intemalization of the receptor, the appearance of an electrical current, a flux or an influx of ions.

6. A method according to claim 4 or 5, comprising contacting a test compound with a cell expressing a xanthurenic acid receptor, in the presence of a ligand of said receptor, measuring a biological or pharmacological effect characteristic of a binding to the xanthurenic acid receptor and comparing the measured effect with that obtained in the absence of the test compound.

7. A method according to anyone of the preceding claims, wherein the binding of the test compound is demonstrated by gel migration, electrophoresis, FRET, SPA or by measuring the displacement of a labelled ligand by the test compound.

**Patentansprüche**

1. In vitro Verfahren zur Durchmusterung, Identifizierung oder Charakterisierung von Verbindungen, die die Aktivität des Systems modulieren, das Xanthurensäure als Mediator verwendet, umfassend das in vitro oder ex vivo Inkontaktbringen einer Testverbindung mit einer Zelle, die einen natürlichen Xanthurensäure-Rezeptor exprimiert, oder einem Membranpräparat, das einen natürlichen Xanthurensäure-Rezeptor aufweist und von einer besagten Rezeptor exprimierenden Zelle abstammt, und den Nachweis einer Bindung der Testverbindung an besagten Rezeptor.

2. Verfahren gemäß Anspruch 1, umfassend das Inkontaktbringen einer Testverbindung mit besagter Zelle oder besagtem Membranpräparat in Gegenwart eines markierten Liganden des besagten Rezeptors und den Nachweis einer Bindung der Testverbindung durch Bestimmen der Verdrängung der Bindung des markierten Liganden.

3. Verfahren gemäß Anspruch 2, **dadurch gekennzeichnet, dass** der markierte Ligand radiomarkierte Xanthurensäure ist.

4. Verfahren gemäß einem der vorhergehenden Ansprüche, **dadurch gekennzeichnet, dass** die Bindung der Testverbindung an den Rezeptor mit Hilfe eines biologischen oder pharmakologischen Effekts nachgewiesen wird.

5. Verfahren nach Anspruch 4, **dadurch gekennzeichnet, dass** der gemessene biologische oder pharmakologische Effekt die Expression eines oder mehrerer zellulärer Proteine, die Expression eines Rezeptors, die Aktivierung eines Gens, die Internalisierung des Rezeptors, das Auftreten eines elektrischen Stroms, eines lonenstroms oder eines loneneinstroms ist.

6. Verfahren nach Anspruch 4 oder 5, umfassend das Inkontaktbringen einer Testverbindung mit einer Zelle, die einen Rezeptor für Xanthurensäure exprimiert, in Gegenwart eines Liganden des besagten Rezeptors, das Messen eines biologischen oder pharmakologischen Effekts, der für eine Bindung an den Xanthurensäure-Rezeptor charakteristisch ist, und den Vergleich des gemessenen Effekts mit demjenigen Effekt, der in Abwesenheit der Testverbindung erhalten wird.

7. Verfahren gemäß einem der vorhergehenden Ansprüche, **dadurch gekennzeichnet, dass** die Bindung der Testverbindung mittels Migration auf einem Gel, Elektrophorese, FRET SPA oder durch Bestimmen der Verdrängung eines markierten Liganden durch die Testverbindung nachgewiesen wird.

Figure 1

**Figure 2A et 2B**

**Figure 3**

**Figure 4**

**Figure 5**

**Figure 6**

0.1 – 0.5 nmoles /g

0.5 – 1.0 nmoles /g

0.5 – 1.0 nmoles /g

> 1 nmoles /g

**Figure 7**

**Figure 8**

**Figure 9**

temps (min.)

|  | LT | LNS | LS |
|---|---|---|---|
| YMAX | 9564 | 5865 | 3482 |
| K | 0.6168 | 0.8860 | 0.5397 |
| Demie-vie | 1.124 | 0.7824 | 1.284 |
| Std. Erreur |  |  |  |
| YMAX | 232.6 | 145.6 | 210.4 |
| K | 0.08527 | 0.1801 | 0.1675 |
| Ajustement de courbe. |  |  |  |
| Degrés de liberté | 24 | 25 | 19 |
| R² | 0.9225 | 0.9049 | 0.7541 |

**FIGURE 10**

Figure 11

| | T | NS | S |
|---|---|---|---|
| BMAX | 32.01 | 76.67 | 6.960 |
| KD | 1.9210e-006 | 8.8370e-006 | 7.4310e-007 |
| Std. Erreur | | | |
| BMAX | 7.059 | 76.23 | 1.237 |
| KD | 5.9230e-007 | 9.6130e-006 | 2.4980e-007 |
| Ajustement de courbe. | | | |
| Degrés de liberté | 19 | 19 | 12 |
| R² | 0.9810 | 0.9779 | 0.9642 |

**Figure 12**

2 sites :
IC 50 $_1$ = 300 nM
IC 50 $_2$ = 57.3 µM
R = 0.97

**Figure 13**

Liaison totale
Liaison non spécifique
Liaison spécifique

**Figure 14**

**Figure 15**

**Figure 16**

EC 50 = 33.4 µM
R = 0.98

**Figure 17**

- liaison Totale
- liaison Non Spécifique
- liaison Spécifique

|  | T | NS | S |
|---|---|---|---|
| BMAX | 867.9 | 410.6 | 581.8 |
| KD | 9.1610e-006 | 2.3220e-005 | 7.5600e-006 |
| Std. Erreur |  |  |  |
| BMAX | 50.18 | 609.6 | 33.57 |
| KD | 9.3210e-007 | 3.5760e-005 | 8.2100e-007 |
| Ajustement de courbe Degrés de liberté | 25 | 16 | 25 |
| R² | 0.9942 | 0.9929 | 0.9922 |

**Figure 18**

1 site :
IC 50 = 2 µM
R = 0.98

2 sites :
IC 50 $_1$ = 1.02 µM
IC 50 $_2$ = 114.4 µM
R = 0.99

**Figure 19**

**FIGURE 20**

**Figure 21**

**Figure 22**

Pipette d'enregistrement

$$V_m = -V_p$$

Application des ligands

X

R

K Cl     K Cl

**Figure 23**

EP 1 402 270 B1

**Figure 24**

**Figure 25.**

**Figure 26.**

**Figure 27**

**Figure 28**

**Figure 29**

**FIGURE 30**

**Figure 31**

Transport ND-1089

Figure 32

Transport ND-1089

Figure 33

Figure 34

## DOPAMINE

**Figure 35**

## GLUTAMATE

**Figure 36**

Figure 37

Figure 38

**Figure 39**